(19) Europäisches Patentamt / European Patent Office / Office européen des brevets

(11) **EP 3 911 950 B1**

(12) **EUROPEAN PATENT SPECIFICATION**

(45) Date of publication and mention of the grant of the patent:
**07.09.2022 Bulletin 2022/36**

(21) Application number: **20701900.1**

(22) Date of filing: **17.01.2020**

(51) International Patent Classification (IPC):
**G01N 33/49** (2006.01)   **G01N 27/74** (2006.01)
**B01L 3/00** (2006.01)   **B03C 1/02** (2006.01)

(52) Cooperative Patent Classification (CPC):
**B01L 3/502761; B03C 1/0332; B03C 1/288; G01N 33/491; B01L 2200/0668; B01L 2300/0645; B01L 2400/043; B03C 2201/18; B03C 2201/26; G01N 27/745**

(86) International application number:
**PCT/IB2020/050376**

(87) International publication number:
**WO 2020/148718 (23.07.2020 Gazette 2020/30)**

(54) **APPARATUS FOR THE QUANTIFICATION OF BIOLOGICAL COMPONENTS DISPERSED IN A FLUID**

VORRICHTUNG ZUR QUANTIFIZIERUNG VON IN EINEM FLUID DISPERGIERTEN BIOLOGISCHEN KOMPONENTEN

APPAREIL DE QUANTIFICATION DE COMPOSANTS BIOLOGIQUES DISPERSÉS DANS UN FLUIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.01.2019 IT 201900000821**

(43) Date of publication of application:
**24.11.2021 Bulletin 2021/47**

(73) Proprietor: **Politecnico di Milano**
**20133 Milano (IT)**

(72) Inventors:
• **BERTACCO, Riccardo**
**21100 Varese (IT)**
• **FIORE, Gianfranco Beniamino**
**20129 Milano (IT)**
• **FERRARI, Giorgio**
**21019 Somma Lombardo (Varese) (IT)**
• **GIACOMETTI, Marco**
**20137 Milano (IT)**
• **MILESI, Francesca**
**24010 Roncobello (Bergamo) (IT)**
• **COPPADORO, Lorenzo Pietro**
**20122 Milano (IT)**
• **GIULIANI, Enrico**
**41126 Modena (IT)**

(74) Representative: **Tana, Maria Gabriella et al**
**Praxi Intellectual Property S.p.A.**
**Via Mario Pagano, 69/A**
**20145 Milano (IT)**

(56) References cited:
**US-A1- 2002 150 886   US-A1- 2011 117 577**
**US-B1- 6 204 656**

• **SANGMOO JEONG ET AL: "Integrated Magneto-Electrochemical Sensor for Exosome Analysis", ACS NANO, vol. 10, no. 2, 25 January 2016 (2016-01-25), pages 1802-1809, XP055433989, US ISSN: 1936-0851, DOI: 10.1021/acsnano.5b07584**
• **GIACOMETTI M ET AL: "On-Chip Magnetophoretic Concentration of Malaria-Infected Red Blood Cells and Hemozoin Nanocrystals", 2018 IEEE INTERNATIONAL MAGNETIC CONFERENCE (INTERMAG), IEEE, 23 April 2018 (2018-04-23), page 1, XP033425505, DOI: 10.1109/INTMAG.2018.8508846**

**Description**

The present invention relates to an apparatus for the quantification of biological, cellular and non-cellular components dispersed in a sample of biological fluids (blood, urine, saliva, sweat, etc.) or in a sample of fluids extracted from biological solids (e.g. faeces). This quantification is performed by magnetophoretic separation and concentration of the components of interest from the rest of the sample and impedimetric detection of the quantity of these components. For the purpose of the present description, "cellular biological components", or simply "cellular components", means biological corpuscles larger or comparable in size to that of the cells, such as for example blood corpuscles (e.g. red blood cells, white blood cells and platelets), the corpuscles contained in urine, pathogens such as bacteria and the eggs of certain parasites. "Non-cellular biological components", or simply "non-cellular components" are understood to be those corpuscles of biological origin that are smaller in volume than cells but larger than individual molecules. Such non-cellular components may be, for example, the crystals of certain substances that develop under particular pathological conditions, such as, for example, the hemozoin crystals produced by the plasmodium of malaria within infected erythrocytes.

In general, the apparatus of the present invention is aimed at the quantification of all those biological components dispersed in a fluid that have different magnetic properties with respect to the medium in which they are suspended. For the purpose of the present description, it is specified that this category comprises those biological components which, following a specific transformation, for example due to a pathological condition, assume a peculiar magnetic behaviour that allows their magnetophoretic separation from their counterparts under physiological conditions. More particularly, the present invention relates to an apparatus that allows and foresees the spatial isolation and concentration of one or more cellular and non-cellular biological components dispersed in a biological fluid (e.g. blood, urine, sweat, saliva, etc.) or in a fluid extracted from a solid biological sample (e.g. faeces), exploiting the differences between the magnetic properties of said components and the magnetic properties of the other components of no interest. Once the separation and concentration have taken place, the apparatus of the present invention foresees, therefore, that the quantification of such cellular and non-cellular biological components takes place through the measurement of the impedance variation between two or more electrodes placed near the concentration zones.

By way of example, one of the fields of application of the apparatus of the present invention can be implemented and realised in such a way as to be used for the detection and quantification of parasite eggs (e.g. schistosoma mansoni) in infested faeces, in that these eggs have a paramagnetic behaviour distinct from the typically diamagnetic behaviour of an aqueous solution in which they can be resuspended [S. Karl et al., PLOS Neglected Tropical Diseases, 7(5), 2219 (2013)].

Another field of application of the present invention relates to, then, the diagnosis of all those pathologies that cause an alteration of the magnetic properties of one or more types of blood cellular components and/ or give rise to the formation of substances with magnetic properties different from plasma, said substances being absent or in different concentrations under physiological conditions. In particular, pathologies are known that cause the alteration of the magnetic properties of erythrocytes, or red blood cells, such as malaria and poisoning that cause an increase in methemoglobinemia. For example, in the case of malaria, it is known that the plasmodium, during the malarial pathogenesis, produces a particular substance to which mention was made above, which takes the name of hemozoin and is a paramagnetic substance. In particular, hemozoin is produced in the form of crystals that accumulate in the infected erythrocytes, making them paramagnetic with respect to the medium in which they are dispersed, that is, plasma. Furthermore, in the non-early phases of malaria, the membrane of the infected red blood cells breaks down, giving rise to the release of the hemozoin crystals in the plasma, which is, instead, diamagnetic. Pathologies are, furthermore, known in which it is not the magnetic properties of the cellular components of the blood that vary but, on the contrary, their density. An example of this type is given by sickle-cell anaemia, where, even if the diamagnetism of the red blood cells remains unchanged, their density changes. In this case, by adding a strongly paramagnetic substance to the plasma, such as gadolinium for instance, it can be thought to exploit the magnetic difference between the red blood cells and the gadolinium solution added to the plasma together with the difference in density between diseased red blood cells and healthy red blood cells to obtain the separation and, therefore, to perform the count of the pathological erythrocytes.

At the state of the art, techniques are known for the separation of cellular components of blood, based on the different magnetic behaviour of these components under physiological and pathological conditions. In particular, patent US5985153A describes a device for the separation of cells or other magneto-sensitive biological entities comprising: a substrate, an external magnetic field generator and a microfluidic system for the loading and unloading of blood.

In application WO2010091874 a particular ferromagnetic structure is described, composed of magnetic conduits, capable of attracting magnetic particles in particular points in which magnetic domain walls are located. In all the prior art documents mentioned above, as well as in a part of the scientific literature listed in the bibliography [S. Bhakdi et al., Optimized high gradient magnetic separation for isolation of Plasmodium-infected red blood cells, Malaria Journal 2010, 9:38]; [J. Nam et al., Magnetic Separation of Malaria-Infected Red Blood Cells in Various Developmental Stages, Anal. Chem., 85, 7316-7323 (2013)]; [Ki-Ho Han and A. Bruno Frazier, Paramagnetic capture mode magnetophoretic microseparator for high efficiency blood cell separations, Lab Chip, 6, 265-273 (2006)], only the magnetophoretic separation of the components of interest from the rest of the blood sample is described, and no mention is made of the detection of the number of such components.

In the patent application US20120003687A and in scientific publications [E. Du., et al., Electric Impedance Microflow Cytometry for Characterization of Cell Disease States, Lab Chip. 2013 October 7; 13(19): 3903-3909] and [M., et al. Ibrahim, J. Claudel, D. Kourtiche and M. Nadi, Geometric parameters optimization of planar interdigitated electrodes for bioimpedance spectroscopy, J Electr Bioimp, vol. 4, pp. 13-22, 2013], techniques of impedimetric quantification of cellular components are, instead, described.

US6204656A discloses a measurement cell with a substrate that carries a plurality of sensors and a magnetic concentrator. A time-varying magnetic field is provided by switching the magnetic field on and off. Each of the sensors consists of a pair of electrodes.

An electronic unit measures the impedance between each pair of electrodes.

These techniques have, however, never been used in association with magnetophoretic separation and concentration. Impedimetric detection requires that the volumetric fraction of the components near the electrodes is sufficiently high in order to obtain a signal-to-noise ratio in the output signal that is sufficient to ensure a correct quantification of the separated components. This concentration is usually obtained with microfluidic techniques aimed at the movement of the blood fluid, which considerably increase the degree of complexity of the system and make it unsuitable for use by a non-specialist user, for example the patient himself or herself. The proposed apparatus intends to overcome these difficulties by replacing the separation by microfluidic movement with a system of magnetic concentration and separation of the components of interest on areas of the measurement cell in which the detection electrodes are located.

Again, with reference to the diagnosis of all those pathologies that cause an alteration of the magnetic properties of one or more types of blood components, in order to carry out the measurement, the non-specialised user can dispense on the special support of said cell a drop of blood just taken, optionally diluted and treated with anticoagulant, and then place it in contact with the substrate of said cell on which the concentrator elements and electrodes are housed, in turn placed within an external magnetic field in such a way that a component of the force of gravity opposes the magnetic attraction force directed towards the concentrators. The measurement cell can also be a preassembled measurement cell, with substrate and support appropriately distanced, optionally integrated with a microfluidic system with preloaded fluids that implements only dilution and anticoagulation of the blood drop and transport within the measurement cell. It is obvious that the measurement cell of the apparatus of the present invention can be further integrated with a microfluidic system that carries out not only the functions of dilution and transport of the sample but also the movement of the blood functional to the same separation of the components, even if this embodiment would have a greater complexity than the embodiments that foresee that the separation takes place only by magnetophoresis and impedimetric detection. For a volume of the blood drop taken of the order of about ten microlitres and assuming that the capture of the components of interest takes place at a maximum distance from the concentrators of between 20 and 200 micrometres, the dimensions of the active area for the capture on the substrate must be of the order of a cm$^2$ and, in particular, comprised between 0.1 and 5 cm$^2$. The support and, therefore, the measurement cell, must also have approximately the same dimensions. On these active area values a high concentration of the components of interest is required in order to ensure an adequate signal-to-noise ratio. As will be explained more clearly here below, this concentration can be quantified by a so-called concentration factor $F_c$ which is given, in the absence of sliding of the components parallel to the substrate and assuming that all the components are captured, by the ratio between the active area of the substrate within which the drop containing the components to be quantified is confined, and the area defined by the detection electrodes. In order to have an adequate signal-to-noise ratio in the output signal, the concentration factor $F_c$ should preferably be at least around 100.

The object of the present invention is, therefore, to provide an apparatus that allows the quantification of the components of interest from a quantity of fluid in which they are dispersed comprised between 5 - 500 microL (5-50 microL, in the case wherein the fluid is blood), and produces an output signal with a signal-to-noise ratio such as to allow the detection of biological cellular and non-cellular components with a lower concentration limit up to tens of components per microlitre.

This object is achieved by the apparatus of the present invention, as defined by appended claim 1.

Said at least one concentrator can be a cylinder or a parallelepiped or an element of another shape placed on the substrate, placed at the detection electrodes, and is made of ferromagnetic material. The concentrator generates an intense local field gradient which attracts the components to be quantified, causing them to concentrate in proximity of said concentrator and, therefore, in proximity of the detection electrodes. In this way, by suitably sizing both the concentrator and the detection electrodes, the concentration factor can increase up to the value necessary to obtain an adequate signal-to-noise ratio.

The detection electrodes are placed in proximity of the concentrator elements, while the reference electrodes are placed not in proximity of the concentrator elements. In other words, the reference electrodes are placed in areas without said concentrators. For the purpose of the present description, "detection electrodes are placed in the proximity of the concentrators" means that the detection electrodes are placed in relation to the concentrators in such a way that their distance in the direction perpendicular to the substrate does not exceed 5 $\mu$m and that the projection of the detection electrodes on the substrate plane is contained within the projection of the concentrators on said substrate plane, or, although not contained within the projection of the concentrators on the substrate plane, is not distant from the projection of the concentrators on the substrate in the direction of the width (e.g. the largest dimension) of the concentrators, for a value not exceeding twice the width of the electrodes. The expression "the reference electrodes are placed not in proximity of the concentrators" means, instead, that the detection electrodes are placed with respect to the concentrators in such a way that the distance between the projection of the concentrators on the substrate and the projection of the detection electrodes on the substrate in the direction of the width of the concentrators is equal to at least twice the characteristic width of the concentrators.

In this way, the separate components accumulate selectively on the detection electrodes but not on the reference electrodes, causing a specific change in impedance between the detection electrodes with respect to the spurious impedance that may be recorded between the reference electrodes. The output signal of the impedimetric quantification system is, therefore, proportional to the difference between the impedance variation recorded between the detection electrodes and that between the reference electrodes. From this output signal, it is then possible to estimate the number of components of interest or, equivalently, their concentration, by comparison with an appropriate calibration curve, carried out by means of a processor.

The means of generation of the magnetic field are configured to realise a modulation of the field and relative gradient at the substrate that houses the concentrator elements, that is to produce variations in time of the magnetic field intensity from a minimum value equal to about 10% of the saturation field of the concentrators to a maximum value that must be higher than the saturation field of the magnetic concentrators. As will be explained in greater detail below, if the magnetisation means are made by means of permanent magnets, this modulation can be simply obtained by a linear motion of moving towards /away from the substrate of the magnets. In particular, when the magnets are brought into proximity of the substrate, the components are captured, while when the magnets are moved away the components detach from the concentrators and from the electrodes. In the case of components with resistance greater than that of the liquid, during the approach phase there is a positive increase in resistance measured at the ends of the detection electrodes, with a characteristic time $\tau_C$. Following a sudden moving away of the magnet, on the other hand, the resistance returns to its initial level for the detachment of the components, with another characteristic time $\tau_R$. Selective capture and release activation by modulation of the magnetic field on the substrate allows a more accurate measurement of impedance variation associated with the components. In this way, thanks to selective capture and release activation, spurious variations, i.e. not synchronous with the moving towards/away of the magnets, can be easily identified, thus obtaining a more accurate measurement of the impedance variation effectively associated with the components, as well as an improved subtraction of the background and drifts associated therewith.

The improvement in the sensitivity of the test, linked to an increase in the ratio between the true signal and the one due to false positives, and the possibility of discriminating between different blood cells, is further guaranteed by the fact that the measurement cell can be inclined with respect to the horizontal plane, i.e. with respect to the plane perpendicular to the gravity acceleration vector.

The separation of the components from the matrix in which they are dispersed, in fact, occurs thanks to the competition between the gravitational force and the magnetic attraction force according to modalities that depend on the characteristics of the components and the angle $\alpha$ formed between the gravity acceleration vector and the line perpendicular to the substrate.

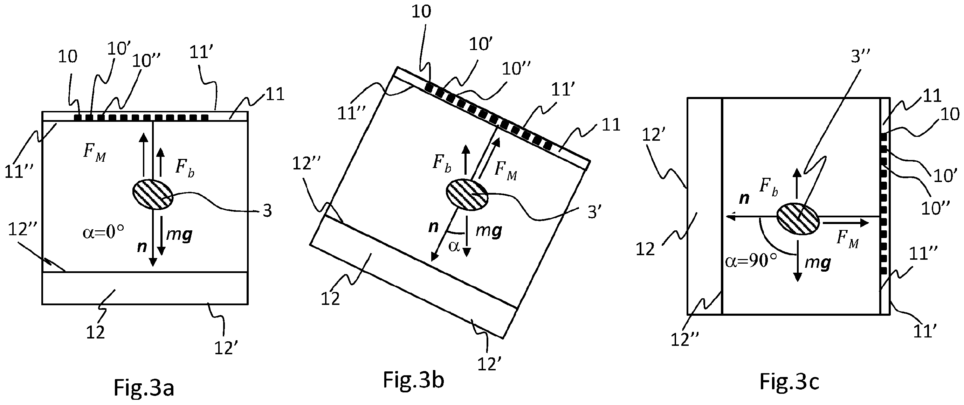

As will be made clearer by the example described here below, the capture efficiency of the captured components varies according to the angle $\alpha$ formed by the line perpendicular to the substrate and by the gravity acceleration vector. For $\alpha = 0°$ (Figure 3a), i.e. when the measurement cell is parallel to the horizontal plane, the magnetic force $F_M$ is antiparallel to the resultant of the weight force (mg) and of that of Archimedes (Fb), or more in general the buoyancy force (Fb), so that, in order to avoid the sedimentation of the components on the support and promote instead an upward motion, a high gradient of the field created by the means for the generation of a magnetic field is necessary. For values of $\alpha$ between 0° and 90°, as shown in Figure 3b, the magnetic force must contrast only the projection along the perpendicular n of the resultant of weight force and force of Archimedes, or, more in general, the resultant of weight force and the buoyancy force (Fb), that is $(mg+F_b)\cos\alpha$. Increasing the angle $\alpha$ decreases the magnetic force threshold beyond which the capture of a given component takes place. It is therefore possible to choose an optimal angle to discriminate the capture of different components present in the sample without acting on the magnetic field generated. In this mode the components fall downwards within the chamber, placed at an angle with respect to the vertical, but during their sliding motion they are attracted towards the substrate, until they sense the field of the concentrators and are therefore captured by them. The extreme case is obtained for $\alpha = 90°$ (Figure 3c), when the weight force and the magnetic force are not in competition, but act along orthogonal axes. The components fall downwards but at the same time are attracted horizontally towards the substrate by the field of the magnet. In this configuration any paramagnetic component is attracted towards the substrate, without any threshold that allows discrimination between components with different magnetic momentum. What varies, however, is the capture efficiency, since the quantity of components captured depends on the intensity of the horizontal attraction force that makes them deviate from the vertical fall motion. For the reasons described above, the measurement cell is fixed in an inclined position of an angle $\alpha$ between the line perpendicular to the substrate of said cell and the gravity acceleration vector comprised between 0° and 180°.

The overall capture efficiency when $\alpha > 0°$, does not depend only on the local capture of the components present within a capture volume in proximity of each concentrator. The same downward sliding motion induced by gravity gradually causes the components above to transit in proximity of the concentrators, thus allowing an increase in the capture efficiency, defined as the number of captured components per unit of volume of liquid in which they are suspended. By shaping the containment ring, as well as the geometry of concentrators and electrodes, so as to convey geometrically the components on the active capture area during their sliding motion induced by gravity, the capture efficiency can therefore be further improved.

A second object of the present invention is, then, to provide an apparatus for the quantification of cellular and non-cellular components that is able to discriminate between different components.

For this purpose, the measurement cell can have a variable inclination instead of a fixed one, and the apparatus of the present invention can comprise a mechanical system configured to make this inclination vary between 0° and 180°.

In other words, the apparatus of the present invention can comprise a mechanical system configured to vary between 0° and 180° the angle $\alpha$ formed between the perpendicular to the substrate of the measurement cell in which the fluid sample is collected and the gravity acceleration vector.

In addition, even the same time modulation of the magnetic field mentioned above and the consequent measurement of capture and release times, which are characteristic of the components to be quantified, provides additional information for the identification of the nature of the same captured components.

Even if, as mentioned above, the apparatus of the present invention is applicable to the diagnosis of any pathology that is the cause of a variation in the magnetic properties of one or more biological components, among the various pathologies for the diagnosis of which it is possible to use the apparatus of the present invention, malaria is of particular interest, in that the diagnostic devices for this type of pathology, present today on the market, have some limitations that make them not always easy to use in particularly disadvantaged contexts, such as those typical of endemic areas, often located in developing countries. The most sensitive method currently available for the diagnosis of malaria is based, in fact, on gene recognition of the various plasmodium strains by PCR (Polymerase Chain Reaction). This type of method is particularly complex and delicate and, therefore, difficult to apply in non-technologically advanced contexts. Moreover, PCR is not a pan-plasmodium method, but is targeted towards specific strains and therefore subject to the problems deriving from continuous mutations of the plasmodium. The "thin smear and/or thick drop" method, on the other hand, which consists of counting the red blood cells infected by plasmodium in a drop of blood under an optical microscope, while not requiring complex instrumentation, requires highly expert personnel and involves a certain variability in the interpretation of the results, as well as long analysis times. Rapid tests (RDT) based on antibody-antigen interaction, on the other hand, are characterised by such low sensitivity as to prevent their use for early diagnosis. In addition, due to the latent presence of the antigen in patients' bodies in the endemic area, methods based on antibody-antigen interaction result in a high number of false positives.

A third object of the present invention is, therefore, to provide an apparatus that also allows the early diagnosis of malaria, whether pan-plasmodium, which has an adequate sensitivity and is simple and economical enough to be used even in those areas where the economic means available do not allow the use of complex instrumentation and specialised personnel.

This object is achieved by the apparatus of the present invention, since the latter is able to carry out both magnetic separation and quantification of infected erythrocytes and direct detection of free hemozoin crystals in plasma. The quantification of infected erythrocytes makes it possible to obtain a direct evaluation of the parasitaemia, which is normally quantified by calculating the ratio between infected erythrocytes and healthy erythrocytes, optionally also in the early phase of the disease, before the completion of the first plasmodium reproduction cycle (48-72 hours). The direct detection of hemozoin crystals is, instead, particularly useful in the non-initial phases of the disease, such as, for example, at the time of the first fever attack, since, in these phases, the erythrocytes have already undergone membrane rupture, and the only thing actually quantifiable in circulation is free hemozoin.

These and further objects of the present invention will be made clearer by the reading of the following detailed description of some preferred embodiments of the present invention by way of a non-limiting example of the more general concepts claimed, as well as the examples concerning experimental tests carried out on the present invention.

The following description refers to the accompanying drawings, in which:

- Figure 1 is a frontal view of a detail of the apparatus according to the present invention, said detail comprising the measurement cell and the means of magnetisation;
- Figure 2 is a lateral view of the apparatus according to the present invention;
- Figure 3a is a frontal view of a detail of the apparatus according to the present invention, said detail comprising the central part of the measurement cell and the means of magnetisation, with a vector diagram of the forces for $\alpha = 0°$;

- Figure 3b is a frontal view of a detail of the apparatus of the present invention, said detail comprising the central part of the measurement cell and the means of magnetisation, with a vector diagram of the forces for a generic angle $\alpha$ comprised between 0 and 90°;
- Figure 3c is a frontal view of a detail of the apparatus of the present invention, said detail comprising the central part of the measurement cell and the means of magnetisation, with a vector diagram of the forces for $\alpha$ =90°;
- Figure 4a is an exemplifying diagram of the measurement method implemented by the apparatus of the present invention through modulation of the field generated by the means for the generation of said field, which shows the approach of the means to the measurement cell;
- Figure 4b is an exemplifying diagram of the measurement method implemented by the apparatus of the present invention through modulation of the field generated by the means for the generation of said field, which shows the moving away of the means in relation to the measurement cell;
- Figure 5 shows the shape of the signal relative to the percentage variation of the resistive component of the impedance after the moving towards and away of the means of magnetisation;
- Figure 6 is an exemplifying diagram of the positioning of the detection and reference electrodes with respect to the concentrators, in a first embodiment of the present invention;
- Figure 7a shows a section of the measurement cell in a first embodiment of the apparatus of the present invention, said section being along a plane perpendicular to the greater dimension of said at least one concentrator;
- Figure 7b shows a detail of the section shown in Figure 7a, relative to said at least one concentrator;
- Figure 7c shows a detail of the section shown in Figure 7a, relative to said at least one pair of detection electrodes;
- Figure 8 is a view from above of the detail of a first embodiment of the apparatus of the present invention, said detail being constituted by the substrate of the measurement cell;
- Figure 9 is a view from above of a detail constituted by the measurement cell of a second embodiment of the present invention;
- Figure 10 shows the trend of the differential percentage variation of the resistive component of the impedance, between the detection electrodes and the reference electrodes, in a second embodiment of the present invention, for $\delta$=40 microns and $\alpha$=90° (i) as a function of the equivalent parasitaemia level of a sample of red blood cells treated in such a way as to make them paramagnetic with respect to plasma (solid symbols and unbroken line) and (ii) for a sample of only healthy red blood cells that provides an indication of the signal due to false positives (dotted line);
- Figure 11 shows the trend of the signal for an equivalent parasitaemia of 0.5% in the case of $\alpha$ =90° and as the height ($\delta$) of the measurement cell varies;
- Figure 12 shows the trend of the ratio between true signal (in the case of an equivalent parasitaemia of 0.5%, $\delta$=40 microns) and spurious signal (due to false positives or fluctuations), for different values of the angle $\alpha$;
- Figure 13 is a view from above of a detail constituted by an unit of the measurement cell of a third embodiment of the present invention;
- Figure 14 is a view from above of a detail constituted by two units of the measurement cell of a third embodiment of the present invention
- Figure 15 is a view from above of a detail constituted by an unit of the measurement cell of a fourth embodiment of the present invention; and
- Figure 16 is a view from above of the detail of a third and fourth embodiment of the apparatus of the present invention, said detail being constituted by the substrate of the measurement cell.

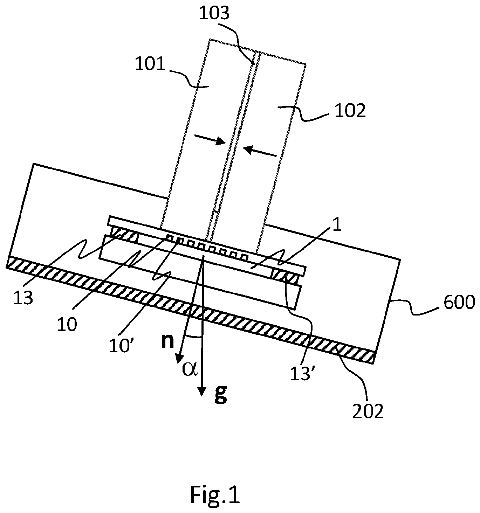

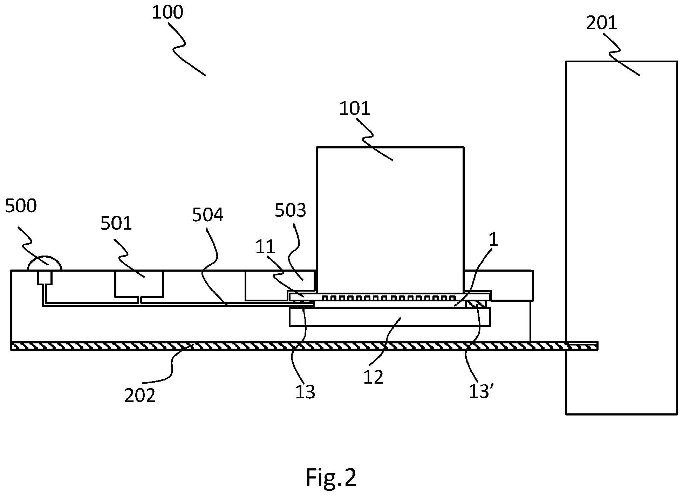

Referring to Figures 1, 2, 3a, 3b and 3c, an embodiment of the apparatus of the present invention (100), intended for the quantification of the erythrocytes infected by the plasmodium of malaria and hemozoin crystals, comprises:

- a measurement cell (1) comprising:

  • a plurality of detection electrodes (4, 4', 5, 5', 6, 6', 34, 34', 84, 84', 94, 94', 184, 184', 194, 194');
  • at least one concentrator (10, 10', 10, 14, 14', 14", 15), said at least one concentrator (10, 10', 10", 14, 14', 14", 15) being configured to magnetically attract the components (3, 3', 3") to be quantified and concentrate said components on the detection electrodes (4, 4', 5, 5', 6, 6', 34,34', 84, 84', 94,94', 184, 184', 194, 194'), said detection electrodes (4,4', 5, 5', 6, 6', 34, 34', 84, 84', 94, 94', 184, 184', 194, 194') being placed in proximity of said at least one concentrator (10, 10', 10", 14,14', 14", 15);
  • at least one pair of reference electrodes (7, 7', 8,8', 9, 9', 37,37', 64, 64', 74, 74', 164, 164', 174') for each pair of detection electrodes (4, 4', 5, 5' 6, 6', 34, 34', 84, 84', 94, 94', 184, 184', 194, 194'), said reference electrodes (7, 7', 8, 8', 9, 9', 37, 37', 64, 64', 74, 74', 164, 164', 174') being placed not in proximity of said at least one concentrator (10, 10', 10", 14, 14', 14", 15);
  • a substrate (11) configured for the housing of the detection electrodes (4, 4', 5, 5', 6, 6', 34, 34', 84, 84', 94, 94',

184, 184', 194, 194'), of the reference electrodes (7, 7', 8, 8', 9, 9', 37, 37', 64, 64', 74, 74', 164, 164', 174') and of the concentrators (10, 10', 10'', 14, 14', 14'', 15), said substrate (11) comprising a first surface (11') facing outside said cell (1) and a second surface (11'') facing inside said cell (1);

- a support (12) comprising a first surface (12') facing outside said cell (1) and a second surface (12'') facing inside said cell (1) and opposite to the second surface (11'') of the substrate (11);
- at least one spacer element (13,13') configured to confine the sample and distance said substrate (11) from said support (12); and
- a mechanical housing (600) configured to house the cell (1) and to make electrical contact between the electrodes (4, 4', 5, 5' 6, 6', 34, 34', 84, 84', 94, 94', 184,184', 194, 194', 7, 7', 8, 8', 9, 9', 37, 37', 64, 64', 74, 74', 164,164', 174') and an electronic board (202);

- an electronic unit (201) connected to said board (202), said electronic unit (201) being configured for the generation of the input signals, amplification of the output signals, measurement of the impedance between the detection electrodes (4, 4', 5, 5', 6, 6', 34, 34', 84, 84', 94, 94', 184, 184', 194, 194'), of the impedance between the reference electrodes (7, 7', 8, 8', 9, 9', 37, 37', 64, 64', 74, 74', 164, 164', 174') or of their difference and communication with a user interface; and
- means for the generation of a magnetic field (101, 102, 103) with gradient that can be modulated in time, said means being configured to generate a magnetic field capable of causing, in combination with concentrators (10, 10', 10'', 14,14', 14'', 15):

- the separation of the components (3, 3', 3'') to be quantified from the rest of the solution; and
- the concentration on the detection electrodes (4, 4', 5, 5', 6, 6', 34, 34', 84, 84', 94, 94', 184, 184', 194, 194') of the components to be quantified (3, 3', 3'');

- a microfluidic system with preloaded fluids that implements dilution and anticoagulation of the blood and transport within the measurement cell (1), said microfluidic system being integrated with the measurement cell (1), and at least partially contained in the mechanical housing (600).
a mechanical system configured to vary the angle α formed between the perpendicular to the substrate of the measurement cell in which the blood sample is collected and the gravity acceleration vector, between 0° and 180°.

The microfluidic system, in turn, comprises

- means for the collection of the blood sample (500) and dilution with preloaded fluids (501) on a cartridge (503); and
- means (504) for transporting the diluted blood sample (500) inside the measurement cell (1).

In order to be able to perform the analysis, the patient's blood drop is, therefore, placed in contact with the inlet of the microfluidic system. It is then sucked in, diluted with preloaded fluids and conveyed into the measurement cell containing the substrate with the electrodes.

The measurement cell can be made with microfabrication techniques on silicon, glass or other polymeric materials, coupled with a suitable microfluidic system made in plastic material.

In the first embodiment of the apparatus of the present invention, intended specifically for use for malaria, the means (101, 102, 103) for the generation of a magnetic field must be able to generate a field which, preferably, has an intensity of at least $10^4$A/m and a macroscopic gradient of its square module of at least $5*10^{14}$ $A^2/m^3$ directed towards or leaving the substrate, respectively in the case of paramagnetic or diamagnetic components with respect to the liquid medium in which they are dispersed. Such means (101, 102,103) may, for example, be realised with a plurality of permanent magnets (101, 102) positioned so that the field generated by said magnets (101, 102) exerts a sufficient force to counteract the resultant of the weight force and that of Archimedes acting on the components of interest at great distance from the substrate (11), thus attracting them towards the surface of the same.

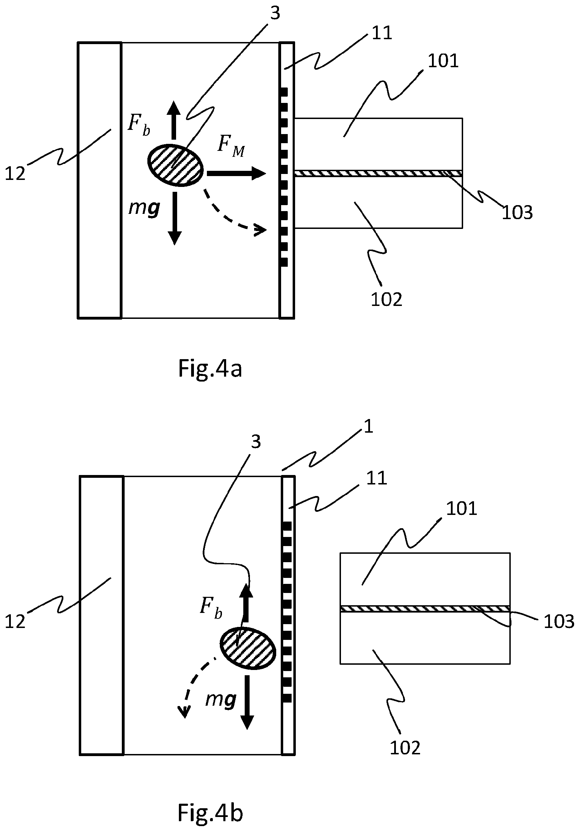

Referring to Figures 4a and 4b, the means for the generation of the magnetic field (101, 102,103) are, in particular, constituted by two permanent magnets (101, 102) of NdFeB N52, in the shape of a parallelepiped with a square base and dimensions 20x20x5 mm, magnetised perpendicularly to the square faces and arranged with the north poles facing. Between them there is a small sheet (103) of mild ferromagnetic material with high magnetic permeability (e.g. Mumetal) that conveys and concentrates the field lines in the symmetry plane of the two magnets (101, 102). At the interface between the small sheet (103) and the air, a symmetrical magnetic field is therefore generated with respect to the separation plane of the magnets (101, 102), with a high gradient having a prevalent component directed orthogonally to the rectangular faces of the magnets (101, 102) placed in contact with or in proximity of the back of the substrate (11). Thus, the field produced is found to have an adequate intensity and gradient to capture the components (3, 3', 3''), in an area of rectangular shape, with a width of about 2 mm and a height of about 16 mm, in order not to include the boundary effects of the magnets (101, 102).

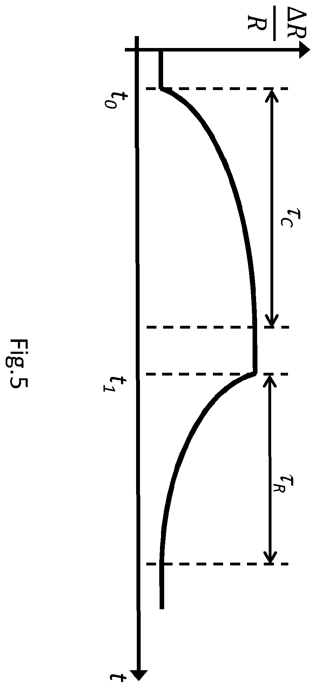

Referring to Figures 4a, 4b and 5, and as mentioned above, the means for the generation of the magnetic field (101, 102, 103) are configured to realise a modulation of the field and relative gradient at the substrate (11) which carries the concentrator elements (10, 10', 10", 14, 14', 14", 15), i.e. to produce variations in the intensity of the magnetic field over time from a minimum value not exceeding 10% of the saturation field of the magnetic concentrators, to a maximum value that must be higher than the saturation field of the magnetic concentrators (10, 10', 10", 14, 14', 14", 15). In the first embodiment of the present invention, the magnetic concentrators are made, for example, in Ni and such modulation can be obtained by a linear motion of moving towards/away from the substrate (11) of the magnets (101, 102). When the means for the generation of the magnetic field (101, 102, 103) are brought in proximity of the substrate (11), the field on the Ni concentrators (10, 10', 10", 14, 14', 14", 15) is sufficient to saturate their magnetisation and therefore allows the capture of the components (3, 3', 3"). When the means for the generation of the magnetic field (101, 102, 103) are moved away the field becomes negligible and the concentrators (10, 10', 10", 14, 14', 14", 15) become demagnetised, so that the components (3,3',3") become detached from the concentrators (10, 10', 10", 14, 14', 14", 15) and from the detection electrodes (4, 4', 5, 5', 6, 6', 34, 34', 84, 84', 94, 94', 184, 184', 194, 194'). As already mentioned, the trend of the resistive component of the impedance measured at the ends of the detection electrodes (4, 4', 5, 5', 6, 6', 34, 34', 84, 84', 94, 94', 184, 184', 194, 194') in the moving towards/away phase is shown in Figure 5. In the approach phase there is a positive $\Delta R$ increase, in the hypothesis of components (3, 3', 3") with resistance greater than that of the liquid, which develops over a characteristic time of capture $\tau_C$. Following a sudden moving away of the means for the generation of the magnetic field (101, 102, 103), the resistance returns to its initial level for the detachment of the components from the detection electrodes (4, 4', 5, 5', 6, 6', 34,34', 84, 84', 94, 94, 184,184', 194,194'), with a characteristic time $\tau_R$.

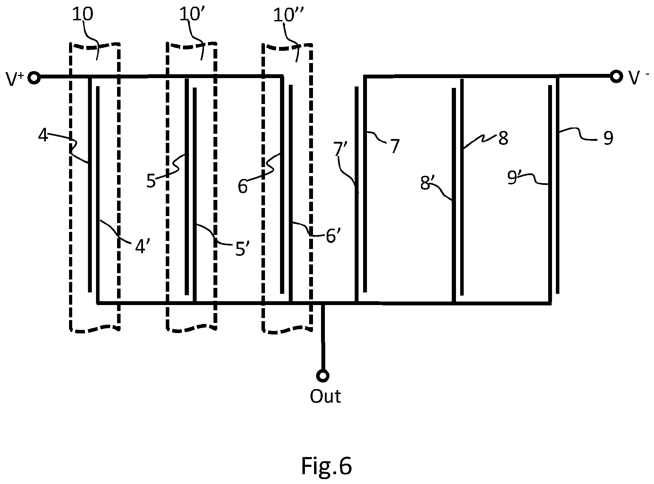

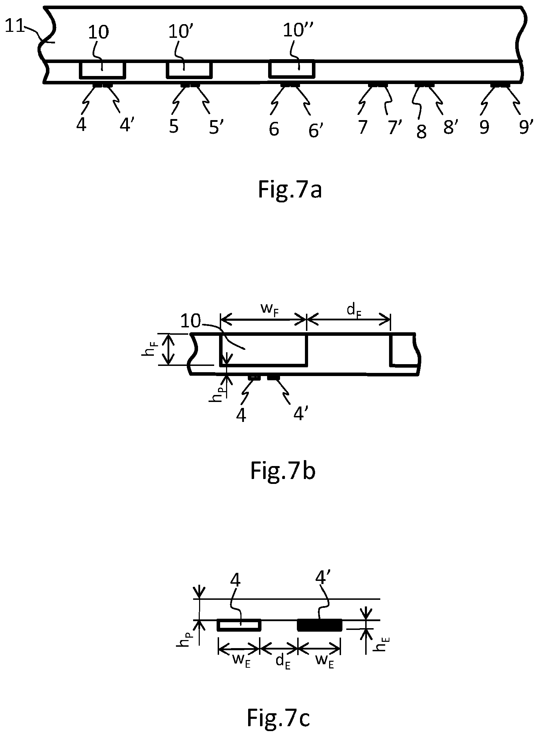

Referring to Figures 6, 7a, 7b and 7c, in the first embodiment of the present invention it is foreseen that each pair of detection electrodes (4, 4', 5, 5', 6, 6') of the measurement cell (1) comprises a first electrode (4, 5, 6) able to receive a first input signal (V+) and a second electrode (4', 5', 6'). Each pair of reference electrodes (7, 7', 8, 8', 9, 9') comprises a first electrode (7, 8, 9) capable of receiving a second input signal (V-) of opposite polarity to the first input signal (V+) and a second electrode (7', 8', 9') connected to the second electrode (4', 5', 6') of each pair of detection electrodes (4, 4', 5, 5', 6, 6'), in a common point from which the output signal (Out) is taken. It is also possible, with obvious modifications of the electronic unit (201), to invert the role of the electrodes and use the common point of the second detection electrode (4', 5', 6') and of the second reference electrode (7', 8', 9') as input signal and the first electrode (4, 5, 6) of the pair of detection electrodes as first output signal and the first electrode (7, 8, 9) of the pair of reference electrodes as second output signal. In all cases the electronic unit (201) will be realised to provide the user with the difference between the impedance of the pair of detection electrodes and the impedance of the pair of reference electrodes in a suitably processed form.

In the first embodiment of the present invention, suitable for the diagnosis of malaria, the concentrators (10, 10', 10") are made of ferromagnetic material, such as Ni, Fe, Co, NiFe, CoFe, etc., and have the shape of a parallelepiped with the largest dimension extending perpendicularly to the plane represented in Figure 3a. In order to ensure a sufficient concentration factor to obtain an adequate signal-to-noise ratio, the dimensions of the concentrators (10, 10', 10") and of the detection electrodes (4,4', 5,5', 6, 6') should preferably be comprised within the ranges listed in Table 1.

| Component | $h_F(\mu m)$ | $w_F(\mu m)$ | $d_F(\mu m)$ | $h_E$ (nm) | $w_E$ ($\mu m$) | $d_E$ ($\mu m$) |
|---|---|---|---|---|---|---|
| i-RBC | 10-50 | 20-100 | 20-100 | 10-300 | 2-6 | 2-6 |
| HC | 10-50 | 10-50 | 10-50 | 10-300 | 1-3 | 1-5 |

Table 1: $h_F$ is the smallest dimension of the base of a concentrator, $w_F$ is the largest dimension of the base of a concentrator and $d_F$ is the distance between a concentrator and the adjacent concentrator. $h_E$ is the smallest dimension of the base of a detection electrode, $w_F$ is the largest dimension of the base of a detection electrode and $d_E$ is the distance between two adjacent electrodes at the same concentrator.

In the first row of Table 1, the concentrator and detection electrode dimension ranges required for correct detection of the erythrocytes infected (i-RBC) by malaria plasmodium are shown; while in the second row of Table 1, the concentrator and detection electrode dimension ranges required for correct detection of free hemozoin (HC) crystals are shown.

The substrate (11) and, therefore, the same cell (1) of the present invention, the structure of the detection electrodes (4,4', 5,5', 6, 6') and of the reference electrodes (7, 7', 8, 8', 9, 9'), can be replicated in four zones into which the substrate (11) is divided, each one divided into two rectangular zones with base equal to 2 mm and height equal to 4 mm. The width of 2 mm is commensurate with the extension of the high magnetic field produced by the magnets in a direction perpendicular to the plane of symmetry. The left part carries the electrodes on the concentrators, and is centred with respect to the plane of symmetry of the magnets described, while the right part has only the reference electrodes for the subtraction of the common mode signal. The subdivision of the active area into several regions with independent readings allows an increase in the ratio between the impedance variation produced by a single component attracted on the detection electrodes and the overall impedance between the electrodes, improving the signal-to-noise ratio in case of low concentrations of components to be detected. Since for each zone an output contact is needed towards the amplifier from which the output signal (Out) is to be emitted, while all the input signals (V+) and (V-) for detection electrodes and reference electrodes require only two contacts, the minimum number of contacts to be formed on the chip is equal to 4+2=6. In order to minimise the impact of possible short circuits between the electrodes during manufacture, three separate contacts can be used for each zone. Consequently, the total number of contacts is 12.

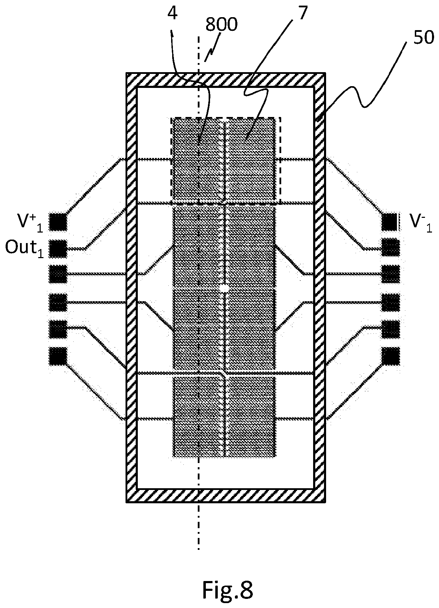

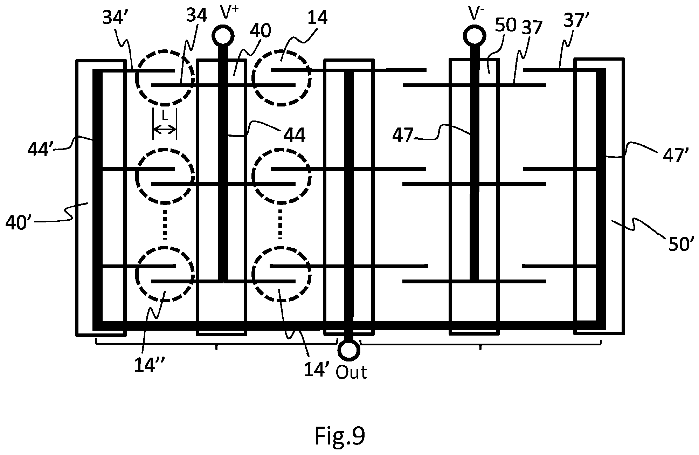

Referring to Figures 8 and 9, a second embodiment of the apparatus of the present invention, again specifically intended for use for malaria, foresees that the apparatus (100) comprises all the same components described above with a single difference related to the configuration of the measurement cell (1). In the second embodiment of the present invention, the measurement cell (1) comprises, in fact, a matrix of ferromagnetic concentrators of cylindrical shape (14,14', 14") uniformly distributed on the substrate (11) according to a square grid. Alternatively, the matrix of concentrators can be arranged according to a hexagonal grid that maximises the packing. In Figure 9, in particular, six pairs of detection electrodes (34, 34') and six pairs of reference electrodes (37, 37') are shown. The first electrode (34) of each pair of detection electrodes (34, 34') is connected to a first input configured to receive the first input signal (V+) via a first connection track (44). The electrodes can be parallel, as shown in Figure 9, or ring-shaped, as in the example described here below, in order to take advantage of the capturing tendency, experimentally detected, on the edges of the cylinders, and thus maximise the occupation of the sensitive area above the electrodes. The first electrode (37) of each pair of reference electrodes (37, 37') is connected to a second input configured to receive the second input signal (V-) via a second connection track (47). Similarly, the second electrode (34') of each pair of detection electrodes (34, 34') is connected to the node from which the output signal (Out) is emitted through a third connection track (44') and the second electrode (37') of each pair of reference electrodes (37, 37') is connected to the node from which said output signal (Out) is emitted through a fourth connection track (47').

Above the first connection track (44), the second connection track (47), the third connection track (44') and the fourth connection track (47') an insulating layer (40, 40', 50, 50') is laid for each track, said insulating layer (40, 40', 50, 50') having dielectric constant and thickness such as to make the impedance between said connection tracks (44, 44', 47, 47') very high so that the effect of this impedance is negligible.

The configuration of the concentrators foreseen by the second embodiment allows a concentration factor to be obtained, at least for $\alpha$=0, even higher than the one obtainable with respect to the first embodiment. For this purpose the dimensions of the concentrators (14, 14', 14") and the detection electrodes (34, 34', 35, 35') must, preferably, be comprised in the ranges listed in Table 2.

| Component | $h_F$ ($\mu$m) | $w_F$ ($\mu$m) | $d_F$ ($\mu$m) | $h_E$ (nm) | $w_E$ ($\mu$m) | $d_E$ ($\mu$m) |
|---|---|---|---|---|---|---|
| i-RBC e HC | 10-50 | 10-50 | 50-200 | 10-300 | 1-3 | 1-5 |

Table 2: $h_F$ is the height of a concentrator, $w_F$ is the diameter of the base of a concentrator and $d_F$ is the distance between one concentrator and the adjacent concentrator. $h_E$ is the smallest dimension of the base of a detection electrode, $w_F$ is the largest dimension of the base of a detection electrode and $d_E$ is the distance between the first finger of a detection electrode and the second finger of said detection electrode.

In Table 2, the ranges of the dimensions of the concentrators and detection electrodes required for proper detection of both erythrocytes infected (i-RBC) by the plasmodium of malaria and free hemozoin crystals (HC) are shown.

With such dimensions, assuming a length L of the straight electrodes shown in Figure 9, equal to 6 $\mu$m, and a

$$F_C = \frac{(d_F+w_F)^2}{L\cdot(2w_E+d_E)}$$

capture efficiency of 100% under conditions of optimal spacing, a geometric concentration factor of about 400 is obtainedReferring to Figure 8, also in the second embodiment of the present invention, the substrate (11) and, therefore, the structure of the detection electrodes (4, 4', 5, 5', 6, 6') and of the reference electrodes (7, 7', 8, 8', 9, 9'), can be replicated in four zones into which the substrate (11) is divided, each one divided into two rectangular zones wherein the left part, or first unit, carries the electrodes on the concentrators, and is centred with respect to the plane of symmetry (800) of the magnets described, while the right part, or second unit, has only the reference electrodes for the subtraction of the common mode signal.

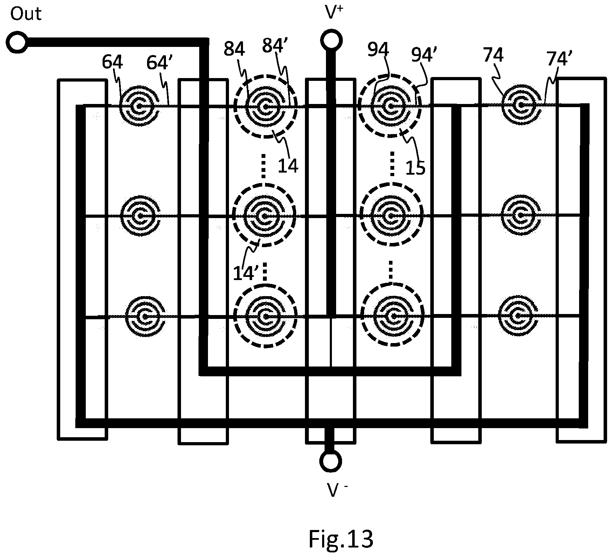

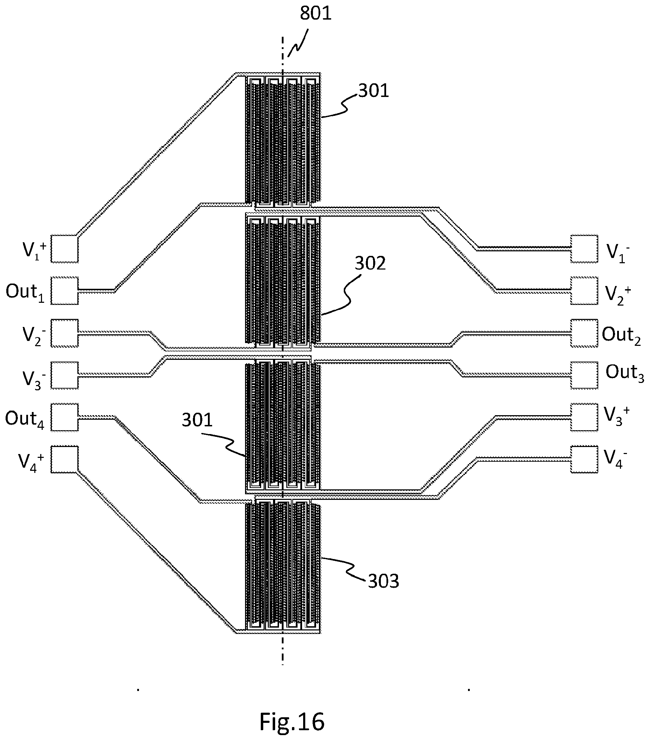

Referring to Figure 13, 14 and 16, a third embodiment of the apparatus of the present invention, again specifically intended for use for malaria, foresees that the apparatus (100) comprises all the same components described above with differences related to the configuration of the measurement cell (1) as in the following.

As in the first and in the second embodiment, the substrate (11) and, thus, the structure of the detection electrodes (84, 84', 94, 94') and of the reference electrodes (64, 64', 74, 74') can be replicated in four zones (301, 302, 303, 304) into which the substrate is divided. The minimum number of contacts to be formed on the chip is always equal to 4+2=6, among which there are four output contacts (one per zone) and two input contacts (one for a first input signal (V+) for all detection electrodes and reference electrodes and one for a second input signal (V-) for all detection and reference electrodes). However, as already mentioned relatively to the first and the second embodiment, in order to minimize the impact of possible short circuits between the electrodes during manufacture, three separate contacts (Out, V+, V-) can be used for each zone. Consequently, the total number of contacts is 12.

Differently from the first and the second embodiment, at least one part of the substrate (11) is centred with respect to the plane of symmetry (801) of the means for the generation of a magnetic field (101, 102, 103), said part carrying:

- the detection electrodes (84, 84', 94, 94') and the reference electrodes (64, 64', 74, 74') interdigitated, or more in general, interlaced, in such a way that said detection and reference electrodes (84, 84', 94, 94', 64, 64', 74, 74') are subject, on average, to the same magnetic field, fluctuations and drifts; and

- the concentrators (10, 10', 10", 14, 14', 14") in proximity of the detection electrodes (84, 84', 94, 94').

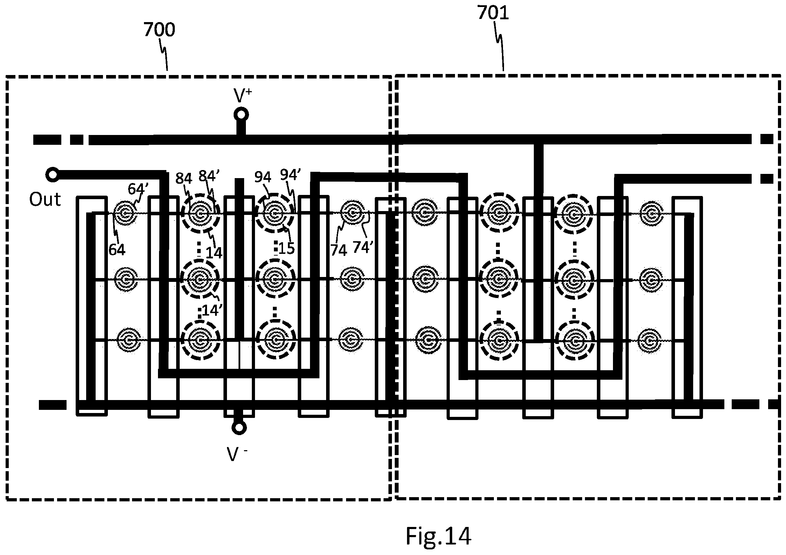

For the purpose of the present description the word "interdigitated" means arranged in alternating, closely packed, single or double stripes. If the detection electrodes (84, 84', 94, 94') and the reference electrodes (64, 64', 74, 74') are interdigitated the subtraction of the background is improved, thus obtaining a more accurate measurement of impedance variation associated with the components to be quantified. In Figure 13, in particular, six pairs of detection electrodes (84, 84', 94, 94'), six pairs of reference electrodes (64, 64', 74, 74') and six concentrators (14,14', 15) are showed. Like in the second embodiment, the concentrators are ferromagnetic and have a cylindrical shape (14,14', 15). The electrodes (64, 64', 74,74', 84, 84', 94, 94') and the concentrators (14,14', 15) are uniformly distributed on the substrate according to a rectangular grid. The concentrators (14, 14', 15) are placed only in proximity of the detection electrodes (84, 84, 94, 94') and not in proximity of said reference electrodes (64, 64', 74, 74'). The detection electrodes (84, 84, 94, 94') and the reference electrodes (64, 64', 74, 74') are arranged in alternating double stripes. More particularly, in each row, both the two pairs of detection electrodes (84, 84', 94, 94') are interposed between a first pair of reference electrodes (74, 74') and a second pair of reference electrodes (64, 64'), and no reference electrodes (74, 74', 64, 64') are interposed between the two pairs of detection electrodes (84, 84', 94, 94'). More generally, the measurement cell comprises at least a first pair of detection electrodes (84, 84', 184, 184') and a second pair of detection electrodes (94, 94', 194, 194'), both the pairs of detection electrodes (84, 84', 184, 184', 94, 94', 194, 194') being interposed between a first pair of reference electrodes (74, 74', 174, 174') and a second pair of reference electrodes (64, 64', 164, 164'), and no reference electrodes (74, 74', 174, 174', 64, 64', 164, 164') being interposed between the first pair of detection electrodes (84, 84', 184, 184') and the second pair of the detection electrodes (94, 94', 194, 194'). The spatial configuration and distribution of the detection electrodes (84, 84', 94, 94') and of the reference electrodes (64, 64', 74, 74') described form a unit (700) that can be replicated to form a second unit (701) and many other identical units, as shoved in Figure 14. In this way a layout is created, where reference electrodes (64, 64', 74, 74') and detection electrodes (84, 84', 94, 94') are closely interdigitated, thus guaranteeing a better subtraction of the spurious fluctuations and, as a consequence, an improvement of the sensitivity of the tests performed using the apparatus (100) of the present invention.

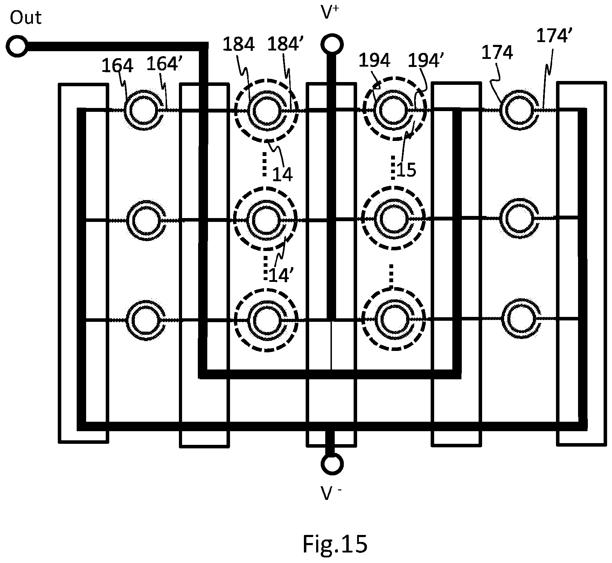

A first electrode (84', 94, 184', 194) of each pair of detection electrodes (84, 84', 94, 94', 184, 184', 194, 194') of the measurement cell (1) is, then, connected to a first input V+, while a first electrode (64, 74') of each pair of reference electrodes (64, 64', 74, 74') is connected to a second input V-. The second electrode (84,94') of each pair of detection electrodes (84, 84', 94, 94') and the second electrode (64', 74) of each pair of reference electrodes (64, 64', 74, 74') are connected to the node wherefrom the output signal (Out) is emitted. The electrodes (64, 64, 74, 74', 84, 84', 94, 94') are ring-shaped. In particular, the end of the one of the electrodes (64, 74, 84, 94) of each pair (64, 64', 74, 74', 84, 84', 94, 94') consists in two open concentric rings. The end of the other (64', 74', 84', 94') of the electrodes (64', 74', 84', 94') of each pair (64, 64', 74, 74', 84, 84', 94, 94') consists, instead, of a straight stretch and of an open ring that surrounds the straight stretch. The stretch is configured to be entered in the inner ring of the end of the first named electrode (64, 74, 84, 94) of the pair and the open ring that surrounds the straight stretch is configured to be entered in the space between the inner and the outer ring of the first named electrode (64, 74, 84, 94) of the pair. Referring to Figure 15 , a fourth embodiment of the apparatus of the present invention, again specifically intended for use for malaria, foresees that the apparatus (100) comprises all the same components described above. The concentrators (14, 14', 15) have the same geometry of the concentrators of the third and of the second embodiment (i.e. cylindrical shape). The measurement cell (1) has the same relative positioning of the detection and reference electrodes (164, 164', 174, 174', 184, 184', 194, 194') detailed above relatively to the third embodiment. The fourth embodiment is characterized by the same layout of the third embodiment, with the reference electrodes (164, 164', 174, 174') and the detection electrodes (184, 184', 194, 194') closely interdigitated and, in particular, arranged in alternating double stripes. The sole difference is the geometry of the electrodes. In the fourth embodiment, the end of the one of the electrodes (164,174,184,194) of each pair (164, 164', 174, 174', 184, 184', 194, 194') consists in an open ring. The end of the other (164', 174', 184', 194') of the electrodes (164', 174', 184', 194') of each pair (164, 164', 174, 174', 184, 184', 194, 194') consists, instead, of a closed ring configured to be entered in the open ring of the first named electrode (164, 174, 184, 194) of the pair. Also in the fourth embodiment, the substrate (11) and, thus, the structure of the detection electrodes (184, 184', 194') and of the reference electrodes (164, 164', 174, 174') can be replicated in four zones (301, 302, 303, 304) into which the substrate is divided. In order to minimize the impact of possible short circuits between the electrodes during manufacture, three separate contacts (Out, V+, V-) can be used for each zone with a the total number of contacts equal to 12. However, the minimum number of contacts to be formed on the chip is six: four output contacts (one per zone) and two input contacts (one for a first input signal (V+) for all detection electrodes and reference electrodes and one for a second input signal (V-) for all detection and reference electrodes.

EXAMPLE

The example described here below relates to the characterisation of an apparatus according to the second embodiment described above, specifically intended for use in malaria. The substrate, according to the structure described in Figure 8, provides for an arrangement of Ni concentrators (with a diameter of 40 microns and a height of 20 microns) arranged according to a hexagonal grid with a centre-centre distance of 160 microns. The electrodes are ring shaped, made of gold with a thickness of 300 nm, width of 3 microns and spacing of 3 microns. The external electrode has an external diameter of 40 microns and is perfectly superimposed on the Ni concentrator, in order to maximise the probability of capture of the components at the electrodes, since specifically on the edges of the cylinders there is the maximum value of the magnetic field and of its gradient. Between the electrodes and the top of the concentrators an insulating layer of SiO2 is interposed with a thickness of 3 microns. The distance between the substrate and the support is determined by a polymeric container ring (50) with a thickness variable between 40 and 500 microns.

The drop of sample containing the components to be analysed is dispensed on the support on which the containment ring is prefabricated, initially placed horizontally, face up. The substrate is made to descend until it presses on the containment ring, creating the seal that allows the fluidic cell to be defined. The cell is housed within a mechanical apparatus that allows variation of the angle $\alpha$, between the perpendicular to the face of the substrate and the gravity acceleration vector, between 0 and 180°. A motorised linear motion allows the magnets to move towards and move away from the substrate in a controlled way and measure correspondingly the resistance variations that are proportional to the concentration of the components. The NdFeB magnets, configured as shown in Figure 1, are able to produce a magnetic field H in proximity of the surface of the substrate towards the measurement cell, i.e. at a distance of 0.5 mm from the surface of the magnets, in the plane of symmetry of the two facing magnets, with module and gradient of the square module equal respectively to $6*10^5$ A m$^{-1}$ and $7*10^{14}$ A$^2$ m$^{-3}$.

For the specific application of malaria diagnostics, the components of interest with paramagnetic properties with respect to plasma are hemozoin crystals and plasmodium-infected red blood cells, which contain some hemozoin crystals. Both hemozoin crystals and red blood cells behave as insulators for input voltage signals applied to them in the range of a few MHz, such as those considered for impedimetric detection. While hemozoin has an absolute positive volumetric magnetic susceptibility, equal to $4.1*10^{-4}$ in units of the S.I., [M. Giacometti et al. APPLIED PHYSICS LETTERS 113, 203703 (2018)], the infected red blood cells have globally a still diamagnetic behaviour. However, the volumetric susceptibility is less negative than that of plasma, so that the difference in susceptibility between infected blood cell and plasma is of the order of $1.8*10^{-6}$, lower than that of hemozoin crystals but sufficient to produce their capture in an appropriate gradient of applied magnetic field H. The difference in susceptibility between the two components makes it possible to discriminate between them, choosing appropriately the value of the magnetic field gradient and the angle $\alpha$ on the basis of an estimate of the forces involved. Assuming a volume of red blood cells $V_{RBC}$ = $9.1*10^{-11}$ cm-3, a density of blood cells $\rho_{RBC}$ = 1.15 g cm$_{-3}$ and a density of plasma $\rho_P$ = 1.025 g cm$_{-3}$, the sum of the weight force and the force of Archimedes on the single blood cell, $F_{gb} = (\rho_{RBC} - \rho_P)V_{RBC}g$ (Figure 3a), results equal to $1.1*10^{-13}$ N.

According to the expression of the magnetic force on a superparamagnetic particle, $$F_M = \frac{1}{2}\mu_0 V_{RBC}\Delta\chi\nabla H^2$$ , assuming a susceptibility difference $\Delta\chi$=$1.8*10^{-6}$ between blood cell and plasma, [K. Han and A. B. Frazier, J. Appl. Phys. 96(10), 5797 (2004)], the value of the H$^2$ gradient necessary to balance $F_{gb}$ in the case of $\alpha$ =0° is of the order of $1*10^{15}$ A$^2$ m$^{-3}$.

A similar estimate can be made for single plasma suspended hemozoin crystal. Assuming an average volume $V_{HC}$ = $2.2*10^{-14}$ cm$^{-3}$, a density $\rho_{HC}$ =1.15 g cm$^{-3}$, and a difference in susceptibility $\Delta\chi$=$4.1*10^{-4}$ with respect to plasma, it is obtained that for hemozoin the value of the H$^2$ gradient necessary to balance $F_{gb}$ is of the order of $1.7*10^{13}$ A$^2$ m$^{-3}$.

From these estimates it results therefore that, for angle $\alpha$=0, where the magnetophoretic force is antiparallel to the resultant of the gravitational force and Archimedes force, the H2 gradient produced by the particular magnets considered ($7*10^{14}$ $A^2$ $m^{-3}$ at the surface of the electrodes of the substrate in the case wherein the magnet is rested on the back of the substrate 0.5 mm thick) is able to attract the hemozoin crystals but not the red blood cells infected by malaria.

Experiments conducted with angle $\alpha$=0 on suspensions of hemozoin crystals in plasma diluted 1:10 with PBS, to simulate the blood dilution expected in the diagnostic test, have in fact shown the possibility of measuring a net change in resistance between the electrodes of the substrate, distinguishable from noise, up to concentrations of hemozoin equal to 1 ng/ml. Assuming that within a red blood cell infected with malaria there are about 18 hemozoin crystals, the concentration detected corresponds to a parasitaemia (percentage ratio between sick and healthy red blood cells) of 0.2%, typical of a patient who has just had a febrile malarial attack.

Under the same conditions (angle $\alpha$ =0) measurements on samples of red blood cells treated with $NaNO_2$ in order to induce the transformation of haemoglobin into paramagnetic methaemoglobin that allows a malaria-infected red blood cell model to be obtained, showed no signal distinguishable from noise. Even if the treated red blood cells (t-RBC) have a difference in magnetic susceptibility with respect to plasma that is double with respect to that of infected blood cells ($\Delta\chi$=3.6*$10^{-6}$), [Nam, Jeonghun, Hui Huang, Hyunjung Lim, Chaeseung Lim, Sehyun Shin. Analytical Chemistry 85, n. 15, 7316-23 (2013)], the H2 gradient necessary to balance the gravitational and buoyancy force ($5*10^{14}$ $A^2$ $m^{-3}$) is very similar to that produced by magnets ($7*10^{14}$ $A^2$ $m^{-3}$), so that any non-ideality or deviation from the values tabulated for the properties of the red blood cells can amply justify the absence of capture and therefore of electrical signal. The situation changes for angles $\alpha$ close to 90°, where the weight and buoyancy force component that opposes the magnetic attraction is practically zero. Since there is no longer a real threshold, both hemozoin and red blood cells are attracted by the macroscopic field gradient towards the substrate, and therefore concentrated on the concentrators by the local gradient during their downward sliding motion.

For the hemozoin the values of the signals detected as a function of concentrations do not vary significantly, since the attraction and concentration were already very effective for $\alpha$ =0. Therefore, a detection limit very similar to that found for $\alpha$ =0, of the order of 1 ng/ml, is obtained for $\alpha$ =90°.

For the treated red blood cells, instead, much more relevant signals are obtained, which allow a limit of detection (LOD) in the order of 0.005% to be obtained, corresponding to about 250 parasites per $\mu$l of blood. At the frequency of the input signals ($V^+$ and $V^-$) of 1 MHz the red blood cells have an insulating behaviour and therefore the variation of the resistive component of the measured $\Delta R/R$ impedance is proportional to the volumetric fraction occupied by them above the concentrator electrodes. It is therefore evident how the greater volume of red blood cells with respect to the hemozoin can lead to signals of greater extent.

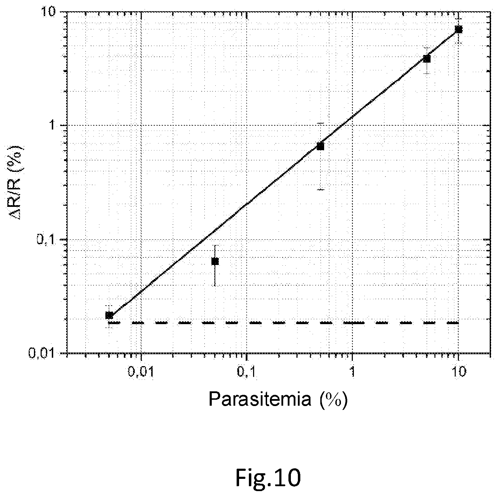

Figure 10 shows with dots and unbroken line the percentage change in resistance measured following the moving away of the magnets from the substrate, i.e. during the release of the red blood cells attracted onto the electrodes, as a function of the equivalent parasitaemia level of the sample. Considering that the diagnostic test involves diluting the patient's blood in PBS (1:10 vol-vol), the sample consisted of a suspension of $NaNO_2$-treated red blood cells in a solution of plasma and PBS (1:10), at decreasing concentrations that can be correlated with the parasitaemia in the following way. Since in 1:10 diluted blood the haematocrit is of the order of 4%, x referring to the percentage volumetric fraction of t-RBC in the sample, the equivalent parasitaemia is x/4.

Also in Figure 10, the dotted line corresponds to the $\Delta R/R$ signal measured with a sample of untreated red blood cells resuspended in plasma and PBS (1:10 vol-vol) with 0.4% haematocrit. This signal is associated partly with false positives and partly with spurious fluctuations induced by the motion of the magnets, but constitutes the lower signal limit below which no significant measurements can be made. As can be seen, the $\Delta R/R$ signal measured follows a substantially linear trend with the parasitaemia of the sample over more than three decades, thus allowing a quantification of the parasitaemia itself. The detection limit, resulting from the intersection of the two curves, is of the order of 0.05%, or about 250 parasites per $\mu$l of blood, and corresponds to the LOD of current rapid diagnostic tests for malaria. In contrast to these, however, the test that is the subject of the present patent application allows a quantitative evaluation of the parasitaemia, which is useful in the phase of diagnosis and of monitoring of the disease following pharmacological treatment.

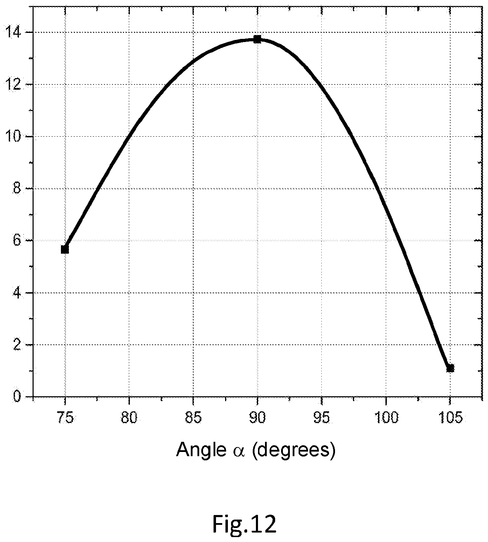

The influence of the angle $\alpha$ on the detection limit has been studied in experiments conducted for angles of 75°, 90° and 105°, as shown in Figure 12. It shows the ratio between the $\Delta R/R$ signal measured at a t-RBC sample with an equivalent parasitaemia of 0.5% and that measured with a sample of untreated red blood cells resuspended in plasma and PBS (1:10 vol-vol) with 0.4% haematocrit, associated with false positives. This ratio, which can be associated with the true/non-specific signal ratio of the test, is maximum for 90°, while it decreases for greater and smaller angles. At 75° the gravity still has a component that opposes the magnetic force, so that the signal of the false positives decreases but also the true signal associable to those treated and in conclusion the ratio decreases. At 105° the gravity contributes to bringing all the healthy and treated blood cells towards the electrodes of the substrate, so that the true signal but also the non-specific one increases. Also in this case however the signal-to-noise ratio decreases. Ultimately, the analysis conducted shows that the 90° angle is preferable for maximising the ratio between true and non-specific signal.

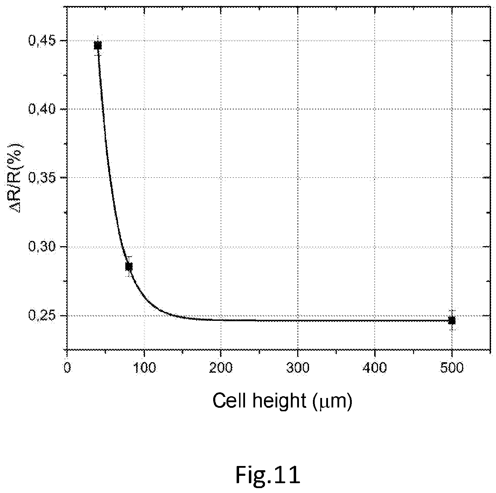

The height of the fluidic measurement cell ($\delta$), defined by the thickness of the spacer element, must itself be optimised to increase the ratio between true and non-specific signal. Figure 11 shows the amplitude of the current signal measured after the second movement away of the magnets, for cell heights of 40, 80 and 500 microns. As can be seen, the net signal decreases with increasing thickness, despite the fact that more blood cells are present in the cell. This is due to the fact that, in the example shown here, the sample is loaded with the cell horizontally ($\alpha$ =0), so that the blood cells have time to settle on the substrate within the typical time (3 minutes) needed to close the cell, make the electrical contacts, stabilise the signal and start the measurement by moving the magnets close. Considering in fact a typical sedimentation rate of 2 micron/ s, even in the case of $\delta$ =500 microns, a thickness of at least 360 microns from the substrate is emptied of blood cells. This means that, at the moment when the sample is moved close, the blood cells are further away than in the case of $\delta$=40 microns, and therefore their capture during the sliding motion is less efficient. This analysis therefore shows that, according to the protocol adopted in this example, the best conditions for increasing the signal correspond to $\delta$=40 microns.

The increase in the cell thickness, and therefore in the number of blood cells contained in it, could instead be exploited if the initial sedimentation of the blood cells was avoided, by appropriate agitation or by initially placing the device at an angle $\alpha$ = 180°. In this way it would be possible to concentrate the blood cells in proximity of the substrate and exploit the sliding motion induced by gravity to move the healthy red blood cells away from the concentrators, capturing instead the diseased ones. Finally, it should be noted how the very dynamic of the signals is rich in information that allows the nature of the signals themselves to be distinguished. For $\alpha$ =90° and $\delta$=40 microns, the dynamic of the signal following the approach of the magnets, corresponding to the capture time $\tau_C$, has the value of 80 s in the case of a false positive signal associated with a sample with 4% haematocrit due to healthy (untreated) blood cells and 150 s in the case of a sample with red blood cells treated with a volumetric fraction corresponding to a parasitaemia of 0.5%. This difference also remains in the case of release $\tau_R$ times of 20 s and 60 s, respectively. Knowing the characteristic dynamics it is therefore possible to identify a spurious impedance variation, e.g. due to false positives or to other fluctuations of the system.

**Claims**

1.  Apparatus (100) for the quantification of biological components (3, 3', 3") in a fluid, comprising:

    - a measurement cell (1) comprising:

        • a plurality of detection electrodes (4, 4', 5, 5', 6, 6', 34, 34', 84, 84', 94, 94', 184,184', 194, 194');
        • at least one concentrator (10,10', 10", 14,14', 14", 15), said at least one concentrator (10, 10', 10", 14, 14', 14", 15) being configured to attract magnetically the components (3, 3', 3") to be quantified and concentrate said components on the detection electrodes (4, 4', 5, 5', 6, 6', 34, 34', 84, 84', 94, 94', 184, 184, 194, 194'), said detection electrodes (4, 4', 5, 5', 6, '6, 34, 34', 84, 84', 94, 94', 184, 184', 194, 194') being placed in proximity of said at least one concentrator (10, 10', 10", 14,14', 14", 15);
        • at least one pair of reference electrodes (7, 7', 8, 8', 9, 9', 37, 37', 64, 64', 74, 74', 164, 164', 174') said reference electrodes (7, 7', 8, 8', 9, 9', 37, 37', 64, 64', 74, 74', 164, 164', 174') being placed not in proximity of said at least one concentrator (10, 10', 10", 14,14', 14", 15);
        • a substrate (11) housing the detection electrodes (4, 4', 5, 5', 6, 6', 34, 34', 84, 84', 94, 94', 184, 184', 194, 194'), the reference electrodes (7, 7', 8, 8', 9, 9', 37,37', 64, 64', 74, 74', 164,164', 174') and the at least one concentrator (10, 10', 10", 14, 14', 14", 15), said substrate (11) comprising a first surface (11') facing the exterior of said cell (1) and a second surface (11") facing the interior of said cell (1);
        • a support (12) comprising a first surface (12') facing the exterior of said cell (1) and a second surface (12") facing the interior of said cell (1) and opposed to the second surface (11") of the substrate (11);
        • at least one spacer element (13, 13'), confining the sample and distancing said substrate (11) from said support (12); and
        • a mechanical housing (600) of the cell (1) creating the electrical contact between the electrodes (4, 4', 5, 5' 6, 6', 34, 34', 84, 84', 94, 94', 184, 184', 194, 194') and an electronic board (202);

    - an electronic unit (201) connected to said board (202), said electronic unit (201) being configured for the generation of input signals, the amplification of the output signals, the measurement of the impedance between the detection electrodes (4, 4', 5, 5', 6, 6', 34, 34', 84, 84', 94, 94', 184, 184', 194, 194'), of the impedance between the reference electrodes (7, 7', 8, 8', 9, 9', 37,37', 64, 64', 74, 74', 164, 164', 174') or of their difference, and the communication with a user interface; and
    - means for the generation of a magnetic field (101, 102, 103) with time-varying intensity and time-varying gradient,

**characterized in that** said means are configured to produce variations in time up to a maximum value of the intensity of the field, said maximum value being higher than the field of saturation of the at least one concentrator (10, 10', 10", 14, 14', 14", 15) and to generate a magnetic field able to cause, in combination with the at least one concentrator (10, 10', 10", 14,14', 14", 15):

- the separation of the components (3, 3', 3") to be quantified from the rest of the solution; and
- the concentration on the detection electrodes (4, 4',5, 5', 6, 6', 34, 34', 84, 84', 94, 94', 184,184', 194,194') of the components (3, 3', 3") to be quantified.

2. Apparatus (100) according to claim 1, wherein said measurement cell (1) comprises at least one pair of reference electrodes (7, 7', 8, 8', 9, 9', 37, 37', 64, 64', 74, 74', 164, 164', 174') for each pair of detection electrodes (4, 4', 5, 5', '6, 34, 34', 84, 84', 94, 94', 184, 184', 194, 194').

3. Apparatus (100) according to claim 1 or 2, wherein the measurement cell (1) is fixed in a slanting position such that the angle between the normal to the substrate (11) of said cell (1) and the gravity acceleration vector is comprised between 0° and 180°.

4. Apparatus (100) according to claim 1 or 2, comprising a mechanical system configured to vary between 0° and 180° the angle between the normal to the substrate (11) of the measurement cell (1) and the gravity acceleration vector.

5. Apparatus (100) according to any one of claims 1 to 4, comprising a microfluidic system with preloaded fluids (501) configured for the dilution of the biological sample and the transport inside the measurement cell (1).

6. Apparatus according to claim 5, wherein said microfluidic system comprises:

- means for the collection of the sample of fluid (500) and dilution with preloaded fluids (501) on a cartridge (503); and
- means (504) for the transport of the sample of fluid (500) diluted inside the measurement cell (1).

7. Apparatus according to claim 5 or 6, wherein said microfluidic system is configured for the anticoagulation of the biological sample, said biological sample containing blood.

8. Apparatus according to claim 4 or 5 wherein the microfluidic system is integrated with the measurement cell (1), said microfluidic system being at least partially contained in the mechanical housing (600).

9. Apparatus (100) according to any one of the preceding claims wherein said at least one spacer element (13, 13') are in the form of an appropriately shaped ring so as to convey the components (3, 3', 3") into the zone of the electrodes.

10. Apparatus (100) according to the preceding claim, wherein said means (101, 102, 103) for the generation of a magnetic field are configured to produce variations in time of said field by means of a linear motion of moving towards/moving away of said means (101, 102, 103) for the generation of a magnetic field at said substrate (11).

11. Apparatus (100) according to any one of the preceding claims, wherein said means (101, 102, 103) for the generation of a magnetic field comprise:

- two permanent magnets (101, 102) in the form of a parallelepiped with square or polygonal base magnetised perpendicularly to the bases; and
- a sheet (103) of mild ferromagnetic material placed between said two permanent magnets (101, 102), between the bases of said magnets having the same polarity, said sheet (103) being configured to concentrate the field lines in the plane of symmetry of the assembly of the two magnets (101, 102).

12. Apparatus (100) according to any one of claims 2 to 11, wherein:

- a first electrode (34) of each pair of detection electrodes (34, 34') of the measurement cell (1) is connected to a first input by means of a first connection track (44);
- a first electrode (37) of each pair of reference electrodes (37, 37') is connected to a second input by means of a second connection track (47);

- a second electrode (34') of each pair of detection electrodes (34, 34') is connected to the node wherefrom an output signal (Out) is emitted by means of a third connection track (44'); and
- a second electrode (37') of each pair of reference electrodes (37, 37') is connected to the node wherefrom said output signal (Out) is emitted by means of a fourth connection track (47');

above each one of said connection tracks (44, 44', 47, 47') being placed an insulating layer (40, 40', 50, 50') with dielectric constant and thickness such as to make the impedance between said connection tracks (44, 44', 47, 47') high in such a way as to make the effect of said impedance negligible.

13. Apparatus (100) according to any one of claims 2 to 12, wherein the substrate (11) is divided in at least two parts, a first part carrying the detection electrodes (4, 4', 5, 5', 6, 6', 34, 34') and the concentrators (10, 10', 10", 14, 14', 14") in proximity of said detection electrodes (4,4', 5, 5', 6, 6', 34, 34') and being centred with respect to the plane of symmetry of the means for the generation of a magnetic field (101, 102, 103), and a second part carrying only the reference electrodes (7, 7', 8, 8', 9, 9', 37, 37') so that they are subject to a lower magnetic field with respect to detection electrodes (4, 4', 5, 5', 6, 6', 34, 34').

14. Apparatus (100) according to any one of claims 2 to 11, wherein at least one part of the substrate (11) is centred with respect to the plane of symmetry (801) of the means for the generation of a magnetic field (101, 102, 103), said part carrying:

- the detection electrodes (84, 84', 94, 94') and the reference electrodes (64, 64', 74, 74') interdigitated in such a way that said detection and reference electrodes (84, 84', 94, 94', 64, 64', 74, 74') are subject, on average, to the same magnetic field, fluctuations and drifts; and

the concentrators (10, 10', 10", 14, 14', 14") in proximity of the detection electrodes (84, 84', 94, 94').

15. Apparatus (100) according to claim 12, wherein the first electrode (4, 5, 6, 34) of said at least one pair of detection electrodes (4, 4', 5, 5', 6, 6', 34, 34') and the second electrode (4', 5', 6', 34') of said at least one pair of detection electrodes (4, 4', 5, 5', 6, 6', 34, 34') are with rectangular section, with base comprised between 10 and 300 nm and height comprised between 1 and 3 $\mu$m.

16. Apparatus (100) according to the preceding claim, wherein the distance between the first electrode (4, 5, 6, 34) of said at least one pair of detection electrodes (4,4', 5, 5', 6, 6', 34, 34') and the second electrode (4', 5', 6', 34') of said at least one pair of detection electrodes (4, 4', 5, 5', 6, 6', 34, 34') is comprised between 1 and 5 $\mu$m.

17. Apparatus (100) according to any of the claims 12 to 16, comprising more than one concentrator (14, 14', 14", 15), wherein said concentrators (14, 14', 14", 15) are cylindrical in shape, the diameter of the base surface of said concentrators (14,14', 14", 15) being comprised between 10 and 50 $\mu$m, the height of said concentrators (14, 14', 14", 15) being comprised between 10 and 50 $\mu$m and the distance between said concentrators (14,14', 14", 15) being comprised between 50 and 150 $\mu$m.

**Patentansprüche**

1. Apparat (100) zur Quantifizierung von biologischen Komponenten (3, 3', 3") in einem Fluid, umfassend:

- eine Messzelle (1), umfassend:

• eine Vielzahl von Detektionselektroden (4, 4', 5, 5', 6, 6', 34, 34', 84, 84', 94, 94', 184,184', 194,194');
• mindestens einen Konzentrator (10, 10', 10", 14, 14', 14", 15), wobei der besagte mindestens eine Konzentrator (10, 10', 10", 14, 14', 14", 15) so konfiguriert ist, dass er die zu quantifizierenden Komponenten (3, 3', 3") magnetisch anzieht und die besagten Komponenten auf den besagten Detektionselektroden (4, 4', 5, 5', 6, '6, 34, 34', 84, 84', 94, 94', 184,184', 194,194') konzentriert, die in der Nähe des besagten mindestens einen Konzentrators (10, 10', 10", 14, 14', 14", 15) angeordnet sind;
• mindestens ein Paar Referenzelektroden (7, 7', 8, 8', 9, 9', 37,37', 64, 64', 74, 74', 164, 164', 174'), wobei die besagten Referenzelektroden (7, 7, 8, 8', 9, 9', 37, 37', 64, 64', 74, 74', 164, 164', 174') nicht in der Nähe des besagten mindestens einen Konzentrators (10, 10', 10", 14, 14', 14", 15) angeordnet sind;
• ein Substrat (11), das die Detektionselektroden (4, 4', 5, 5', 6, 6', 34, 34', 84, 84', 94, 94', 184,184', 194,194'), die Referenzelektroden (7, 7, 8, 8', 9, 9', 37, 37',64, 64', 74, 74', 164,164', 174') und den mindestens einen Konzentrator (10, 10', 10", 14, 14', 14", 15) beherbergt, wobei das besagte Substrat (11) eine erste Oberfläche (11'), die der Außenseite der besagten Zelle (1) zugewandt ist, und eine zweite Oberfläche (11"), die dem Inneren der besagten Zelle (1) zugewandt ist, umfasst;
• einen Träger (12), umfassend eine erste Oberfläche (12'), die der Außenseite der besagten Zelle (1) zugewandt ist, und eine zweite Oberfläche (12"), die dem Inneren der besagten Zelle (1) zugewandt ist und der zweiten Oberfläche (11") des Substrats (11) gegenüberliegt;
• mindestens ein Abstandselement (13, 13'), das die Probe begrenzt und das besagte Substrat (11) von dem besagten Träger (12) distanziert; und
• ein mechanisches Gehäuse (600) der Zelle (1), das den elektrischen Kontakt zwischen den Elektroden (4, 4', 5, 5', 6, 6', 34, 34', 84, 84', 94, 94', 184, 184', 194, 194') und einer elektronischen Platte (202) herstellt;

- eine elektronische Einheit (201), die mit der besagten Platte (202) verbunden ist, wobei die besagte elektronische Einheit (201) für die Erzeugung von Eingangssignalen, die Verstärkung von den Ausgangssignalen, die Messung der Impedanz zwischen den Detektionselektroden (4, 4', 5, 5', 6, 6', 34, 34', 84, 84', 94, 94', 184, 184', 194, 194'), der Impedanz zwischen den Referenzelektroden (7, 7', 8, 8', 9, 9', 37, 37', 64, 64', 74, 74', 164, 164', 174') oder deren Differenz und die Kommunikation mit einer Benutzerschnittstelle konfiguriert ist; und
- Mittel zur Erzeugung eines Magnetfeldes (101, 102, 103) mit zeitlich veränderlicher Intensität und zeitlich veränderlichem Gradienten,

**dadurch gekennzeichnet, dass** die besagten Mittel so konfiguriert sind, dass sie zeitliche Variationen bis zu einem Maximalwert der Intensität des Feldes erzeugen, wobei der besagte Maximalwert höher ist als das Sättigungsfeld des mindestens einen Konzentrators (10, 10', 10", 14, 14', 14", 15), und dass sie ein Magnetfeld erzeugen, das in Kombination mit dem mindestens einen Konzentrator (10, 10', 10", 14, 14', 14", 15) Folgendes bewirkt:

- die Abtrennung der zu quantifizierenden Komponenten (3, 3', 3") vom Rest der Lösung; und
- die Konzentration auf den Detektionselektroden (4, 4', 5, 5', 6, 6', 34, 34', 84, 84', 94, 94', 184, 184', 194, 194') der zu quantifizierenden Komponenten (3, 3', 3").

2. Apparat (100) gemäß Anspruch 1, wobei die besagte Messzelle (1) mindestens ein Paar Referenzelektroden (7, 7', 8, 8', 9, 9', 37, 37', 64, 64', 74, 74', 164, 164', 174') für jedes Paar Detektionselektroden (4, 4', 5, 5', 6, 6', 34, 34', 84, 84', 94, 94', 184, 184', 194, 194') umfasst.

3. Apparat (100) gemäß Anspruch 1 oder 2, wobei die Messzelle (1) in einer Schräglage befestigt ist, so dass der Winkel zwischen der Senkrechten zum Substrat (11) der besagten Zelle (1) und dem Schwerebeschleunigungsvektor zwischen 0° und 180° liegt.

4. Apparat (100) gemäß Anspruch 1 oder 2, umfassend ein mechanisches System, das so konfiguriert ist, dass es den Winkel zwischen der Senkrechten zum Substrat (11) der Messzelle (1) und dem Schwerebeschleunigungsvektor zwischen 0° und 180° variiert.

5. Apparat (100) gemäß einem jeglichen der Ansprüche 1 bis 4, umfassend ein mikrofluidisches System mit vorgeladenen Fluiden (501), die für die Verdünnung der biologischen Probe und den Transport innerhalb der Messzelle (1) konfiguriert sind.

6. Apparat gemäß Anspruch 5, wobei das besagte mikrofluidische System Folgendes umfasst:

- Mittel zur Entnahme der Fluidprobe (500) und Verdünnung mit vorgeladenen Fluiden (501) auf einer Kartusche (503); und
- Mittel (504) für den Transport der Probe des in der Messzelle (1) verdünnten Fluids (500).

7. Apparat gemäß Anspruch 5 oder 6, wobei das besagte mikrofluidische System für die Antikoagulation der biologischen Probe konfiguriert ist, wobei die besagte biologische Probe Blut enthält.

8. Apparat gemäß Anspruch 4 oder 5 wobei das mikrofluidische System mit der Messzelle (1) integriert ist, wobei das besagte mikrofluidische System zumindest teilweise in dem mechanischen Gehäuse (600) enthalten ist.

9. Apparat (100) gemäß einem jeglichen der vorhergehenden Ansprüche, wobei das besagte mindestens eine Abstandselement (13, 13') die Form eines entsprechend geformten Rings besitzt, so dass die Komponenten (3, 3', 3")
in den Bereich der Elektroden befördert werden.

10. Apparat (100) gemäß dem vorhergehenden Anspruch, wobei die besagten Mittel (101, 102, 103) zur Erzeugung
eines Magnetfeldes so konfiguriert sind, dass sie zeitliche Variationen des besagten Feldes mittels einer linearen
Bewegung des Hinbewegens/Wegbewegens der besagten Mittel (101, 102, 103) zur Erzeugung eines Magnetfeldes
an dem besagten Substrat (11) hervorbringt.

11. Apparat (100) gemäß einem jeglichen der vorhergehenden Ansprüche, wobei die besagten Mittel (101, 102, 103)
zur Erzeugung eines Magnetfeldes Folgendes umfassen:

- zwei Permanentmagneten (101, 102) in Form eines Parallelepipeds mit quadratischer oder polygonaler Grundfläche, die senkrecht zu den Grundflächen magnetisiert sind; und
- eine Platte (103) aus mildem ferromagnetischem Material, die zwischen den besagten beiden Permanentmagneten (101, 102) angeordnet ist, zwischen den Grundflächen der besagten Magneten, die die gleiche Polarität
haben, wobei die besagte Platte (103) so konfiguriert ist, dass sie die Feldlinien in der Symmetrieebene der
Anordnung der beiden Magnete (101, 102) konzentriert.

12. Apparat (100) gemäß einem jeglichen der Ansprüche 2 bis11, wobei:

- eine erste Elektrode (34) jedes Paares von Detektionselektroden (34, 34') der Messzelle (1) mit einem ersten
Eingang mittels einer ersten Verbindungsspur (44) verbunden ist;
- eine erste Elektrode (37) jedes Paares von Referenzelektroden (37, 37') mit einem zweiten Eingang mittels
einer zweiten Verbindungsspur (47) verbunden ist;
- eine zweite Elektrode (34') jedes Paares von Detektionselektroden (34, 34') mit dem Knoten verbunden ist,
von dem ein Ausgangssignal (Out) mittels einer dritten Verbindungsspur (44') ausgegeben wird; und
- eine zweite Elektrode (377) eines jeden Paares von Referenzelektroden (37, 37') mit dem Knoten verbunden
ist, von dem das Ausgangssignal (Out) mittels einer vierten Verbindungsspur (47') ausgegeben wird;

wobei über jeder der besagten Verbindungsspuren (44, 44', 47, 47') eine Isolierschicht(40, 40', 50, 50') mit einer
solchen Dielektrizitätskonstanten und Dicke angeordnet ist, dass die Impedanz zwischen den besagten Verbindungsspuren (44, 44', 47, 47') so hoch ist, dass die Wirkung der Impedanz vernachlässigbar ist.

13. Apparat (100) gemäß einem jeglichen der Ansprüche 2 bis 12, wobei das Substrat (11) in mindestens zwei Teile
unterteilt ist, wobei ein erster Teil die Detektionselektroden (4, 4', 5, 5', 6, 6', 34, 34') und die Konzentratoren (10,
10', 10", 14, 14', 14") in der Nähe der besagten Detektionselektroden (4, 4', 5, 5', 6, 6', 34, 34') trägt und die in
Bezug auf die Symmetrieebene der Mittel zur Erzeugung eines Magnetfeldes (101, 102, 103) zentriert sind, und
einen zweiten Teil, der nur die Referenzelektroden (7, 7', 8, 8', 9, 9', 37, 37') trägt, so dass sie einem niedrigeren
Magnetfeld in Bezug auf die Detektionselektroden (4, 4', 5, 5', 6, 6', 34, 34') ausgesetzt sind.

14. Apparat (100) gemäß einem jeglichen der Ansprüche 2 bis 11, wobei mindestens ein Teil des Substrats (11) in
Bezug auf die Symmetrieebene (801) der Mittel zur Erzeugung eines Magnetfelds (101, 102, 103) zentriert ist, wobei
der besagte Teil Folgendes trägt:

- die Detektionselektroden (84, 84', 94, 94') und die Referenzelektroden (64, 64', 74, 74'), die so ineinandergreifen, dass die Detektions- und Referenzelektroden (84, 84', 94, 94', 64, 64', 74, 74') im Durchschnitt demselben Magnetfeld, denselben Fluktuationen und denselben Driften ausgesetzt sind; und

die Konzentratoren (10, 107, 10", 14, 147, 14") in der Nähe der Detektionselektroden (84,847,94,947).

15. Apparat (100) gemäß Anspruch 12, wobei die erste Elektrode (4, 5, 6, 34) des besagten mindestens einen Paares
von Detektionselektroden (4, 4', 5, 5', 6, 6', 34, 34') und die zweite Elektrode (4', 5', 6', 34') des besagten mindestens
einen Paares von Detektionselektroden (4, 4', 5, 5', 6, 6', 34, 34') einen rechteckigen Querschnitt mit einer Grundfläche zwischen 10 und 300 nm und einer Höhe zwischen 1 und 3 $\mu$m besitzen.

16. Apparat (100) gemäß dem vorhergehenden Anspruch, wobei der Abstand zwischen der ersten Elektrode (4, 5, 6,
34) des besagten mindestens einen Paares von Detektionselektroden (4, 4', 5, 5', 6, 6', 34, 34') und der zweiten
Elektrode (4', 5', 6', 34') des besagten mindestens einen Paares von Detektionselektroden (4, 4', 5, 5', 6, 6', 34, 34') zwischen 1 und 5 µm beträgt.

17. Apparat (100) gemäß einem jeglichen der Ansprüche 12 bis 16, umfassend mehr als einen Konzentrator (14, 14', 14", 15), wobei die besagten Konzentratoren (14, 14', 14", 15) zylindrisch geformt sind, der Durchmesser der Grundfläche der besagten Konzentratoren (14, 14', 14", 15) zwischen 10 und 50 µm liegt, die Höhe der besagten Konzentratoren (14, 14', 14", 15) zwischen 10 und 50 µm liegt und der Abstand zwischen den besagten Konzentratoren (14, 14', 14", 15) zwischen 50 und 150 µm liegt.

**Revendications**

1. Appareil (100) pour la quantification de composants biologiques (3, 3', 3") dans un fluide comprenant :

   - une cellule de mesure (1) comprenant :

     • une multitude d'électrodes de détection (4, 4', 5, 5', 6, 6', 34, 34', 84, 84', 94, 94', 184, 184', 194, 194') ;
     • au moins un concentrateur (10, 10', 10", 14, 14', 14", 15), ledit au moins un concentrateur (10, 10', 10", 14, 14', 14", 15) étant configuré pour attire magnétiquement les composants (3, 3', 3") à quantifier et concentrer lesdits composants sur les électrodes de détection (4, 4', 5, 5', 6, 6', 34, 34', 84, 84', 94, 94', 184, 184', 194, 194'), lesdites électrodes de détection (4, 4', 5, 5', 6, 6', 34, 34', 84, 84', 94, 94', 184, 184', 194, 194') étant placées à proximité dudit concentrateur au minimum (10, 10', 10", 14, 14', 14", 15) ;
     • au moins une paire d'électrodes de référence (7, 7', 8, 8', 9, 9', 37, 37', 64, 64', 74, 74', 164, 164', 174, 174') lesdites électrodes de référence (7, 7', 8, 8', 9, 9', 37, 37', 64, 64', 74, 74', 164, 164', 174, 174') étant placées non à proximité dudit concentrateur au minimum (10, 10', 10", 14, 14', 14", 15) ;
     • un substrat (11) logeant les électrodes de détection (4, 4', 5, 5', 6, 6', 34, 34', 84, 84', 94, 94', 184, 184', 194, 194'), les électrodes de référence (7, 7', 8, 8', 9, 9', 37, 37', 64, 64', 74, 74', 164, 164', 174, 174') et le concentrateur au minimum (10, 10', 10", 14, 14', 14", 15), ledit substrat (11) comprenant une première surface (11') tournée vers l'extérieur de ladite cellule (1) et une seconde surface (11") tournée vers l'intérieur de ladite cellule (1) ;
     • un support (12) comprenant une première surface (12') tournée vers l'extérieur de ladite cellule (1) et une seconde surface (12") tournée vers l'intérieur de ladite cellule (1) et opposée à la seconde surface (12") du substrat (11) ;
     • au moins un élément d'espacement (13, 13'), confinant l'échantillon et distançant ledit substrat (11) dudit support (12) ; et
     • un boîtier mécanique (600) de la cellule (1) créant le contact électrique entre les électrodes (4, 4', 5, 5', 6, 6', 34, 34', 84, 84', 94, 94', 184, 184', 194, 194') et une carte électronique (202) ;

   - une unité électronique (201) connectée à ladite carte (202), ladite unité électronique (201) étant configurée pour la génération des signaux d'entrée, l'amplification des signaux de sortie, la mesure de l'impédance entre les électrodes de détection (4, 4', 5, 5', 6, 6', 34, 34', 84, 84', 94, 94', 184, 184', 194, 194'), de l'impédance entre les électrodes de référence (7, 7', 8, 8', 9, 9', 37, 37', 64, 64', 74, 74', 164, 164', 174, 174') ou de leur différence, et la communication avec une interface utilisateur ; et
   - des moyens pour générer un champ magnétique (101, 102, 103) dont l'intensité et le gradient varient dans le temps,

   **caractérisé par le fait que** lesdits moyens sont configurés pour produire une variation dans le temps jusqu'à une valeur maximale de l'intensité du champ, ladite valeur maximale étant supérieure au champ de saturation du concentrateur au minimum (10, 10', 10", 14, 14', 14", 15) et pour générer un champ magnétique capable de provoquer, en combinaison avec le concentrateur au minimum (10, 10', 10", 14, 14', 14", 15) :

   - la séparation des composants (3, 3', 3") à quantifier du reste de la solution ; et
   - la concentration sur les électrodes de détection (4, 4', 5, 5', 6, 6', 34, 34', 84, 84', 94, 94', 184, 184', 194, 194') des composants (3, 3', 3") à quantifier.

2. Appareil (100) selon la revendication 1, où ladite cellule de mesure (1) comprend au moins une paire d'électrodes de référence (7, 7', 8, 8', 9, 9', 37, 37', 64, 64', 74, 74', 164, 164', 174, 174') pour chaque paire d'électrodes de détection (4, 4', 5, 5', 6, 6', 34, 34', 84, 84', 94, 94', 184, 184', 194, 194').

**3.** Appareil (100) selon la revendication 1 ou 2, où la cellule de mesure (1) est fixée dans une position inclinée telle que l'angle entre la normale au substrat (11) de ladite cellule (1) et le vecteur d'accélération de la gravité est compris entre 0° et 180°.

**4.** Appareil (100) selon la revendication 1 ou 2, comprenant un système mécanique configuré pour faire varier entre 0° et 180° l'angle entre la normale au substrat (11) de la cellule de mesure (1) et le vecteur d'accélération de la gravité.

**5.** Appareil (100) selon l'une des revendications 1 à 4, comprenant un système microfluidique avec des fluides préchargés (501) configurés pour la dilution de l'échantillon biologique et le transport dans la cellule de mesure (1).

**6.** Appareil selon la revendication 5, où ledit système microfluidique comprend :

- des moyens pour la collecte de l'échantillon de fluide (500) et la dilution avec des fluides préchargés (501) sur une cartouche (503) ; et
- des moyens (504) pour le transport de l'échantillon de fluide (500) dilué à l'intérieur de la cellule de mesure (1).

**7.** Appareil selon la revendication 5 ou 6, où ledit système microfluidique est configuré pour l'anticoagulation de l'échantillon biologique, ledit échantillon biologique contenant du sang.

**8.** Appareil selon la revendication 4 ou 5, où le système microfluidique est intégré à la cellule de mesure (1), ledit système microfluidique étant au moins partiellement contenu dans le boîtier mécanique (600).

**9.** Appareil (100) selon l'une des revendications précédentes, dans lequel ledit au moins un élément d'espacement (13, 13') se présente sous la forme d'un anneau de forme appropriée de façon à transporter les composants (3, 3', 3") dans la zone des électrodes.

**10.** Appareil (100) selon la revendication précédente, où lesdits moyens (101, 102, 103) pour la génération d'un champ magnétique sont configurés pour produire des variations dans le temps dudit champ au moyen d'un mouvement linéaire de rapprochement/éloignement desdits moyens (101, 102, 103) pour la génération d'un champ magnétique au niveau dudit substrat (11).

**11.** Appareil (100) selon l'une des revendications précédentes, dans lequel lesdits moyens (101, 102, 103) pour la génération d'un champ magnétique comprennent :

- deux aimants permanents (101, 102) en forme de parallélépipède à base carrée ou polygonale magnétisés perpendiculairement aux bases ; et
- une feuille (103) de matériau ferromagnétique doux placée entre lesdits deux aimants permanents (101, 102), entre les bases desdits aimants ayant la même polarité, ladite feuille (103) étant configurée pour concentrer les lignes de champ dans le plan de symétrie de l'ensemble des deux aimants (101, 102).

**12.** Appareil (100) selon l'une des revendications de 2 à 11, où :

- une première électrode (34) de chaque paire d'électrodes de détection (34, 34') de la cellule de mesure (1) est connectée à une première entrée au moyen d'une première piste de connexion (44) ;
- une première électrode (37) de chaque paire d'électrodes de référence (37, 37') est connectée à une deuxième entrée au moyen d'une deuxième piste de connexion (47) ;
- une seconde électrode (34') de chaque paire d'électrodes de détection (34, 34') est connectée au noeud à partir duquel un signal de sortie (Out) est émis par l'intermédiaire d'une troisième piste de connexion (44') ; et
- une seconde électrode (37') de chaque paire d'électrodes de référence (37, 37') est connectée au noeud à partir duquel un signal de sortie (Out) est émis par l'intermédiaire d'une quatrième piste de connexion (47') ;

au-dessus de chacune desdites pistes de connexion (44, 44', 47, 47') étant placée une couche isolante (40, 40', 50, 50') de constante diélectrique et d'épaisseur telles que l'impédance entre lesdites pistes de connexion (44, 44', 47, 47') soit élevée de manière à rendre l'effet de ladite impédance négligeable.

**13.** Appareil (100) selon l'une des revendications de 2 à 12, où le substrat (1) est divisé en au moins deux parties, une première partie portant les électrodes de détection (4, 4', 5, 5', 6, 6', 34, 34') et le concentrateur (10, 10', 10", 14, 14', 14") à proximité desdites électrodes de détection (4, 4', 5, 5', 6, 6', 34, 34') et étant centré par rapport au plan de symétrie des moyens de génération d'un champ magnétique (101, 102, 103), et une seconde partie portant uniquement les électrodes de référence (7, 7', 8, 8', 9, 9', 37, 37') de manière à ce qu'elles soient soumises à un champ magnétique plus faible par rapport aux électrodes de détection (4, 4', 5, 5', 6, 6', 34, 34').

14. Appareil (100) selon l'une des revendications de 2 à 11, où au moins une partie du substrat (11) est centrée par rapport au plan de symétrie (801) des moyens de génération d'un champ magnétique (101, 102, 103), ladite partie portant :

- les électrodes de détection (84, 84', 94, 94') et les électrodes de référence (64, 64', 74, 74') imbriquées de telle sorte que lesdites électrodes de détection et de référence (84, 84', 94, 94', 64, 64', 74, 74') sont soumises, en moyenne, au même champ magnétique, aux mêmes fluctuations et dérives ; et
- les concentrateurs (10, 10', 10", 14, 14', 14") à proximité des électrodes de détection (84, 84', 94, 94').

15. Appareil (100) selon la revendication 12, où la première électrode (4, 5, 6, 34) de ladite paire d'électrodes de détection au minimum (4, 4', 5, 5', 6, 6', 34 , 34') et la seconde électrode (4', 5 ', 6', 34') de ladite paire d'électrodes de détection au minimum (4, 4', 5, 5', 6, 6', 34, 34') ont une section rectangulaire, avec une base comprise entre 10 et 300 nm et une hauteur comprise entre 1 et 3 $\mu$m.

16. Appareil (100) selon la revendication précédente, où la distance entre la première électrode (4, 5, 6, 34) de ladite paire d'électrodes de détection au minimum (4, 4', 5, 5', 6, 6', 34, 34') et la seconde électrode (4', 5', 6', 34') de ladite paire d'électrodes de détection au minimum (4, 4', 5, 5', 6, 6', 34, 34') est comprise entre 1 et 5 $\mu$m.

17. Appareil (100) selon l'une des revendications de 12 à 16, comprenant plus d'un concentrateur (14, 14', 14", 15), où lesdits concentrateurs (14, 14', 14", 15) sont de forme cylindrique, le diamètre de la surface de base desdits concentrateurs (14, 14', 14", 15) étant compris entre 10 et 50 $\mu$m, et la distance entre lesdits concentrateurs (14, 14', 14", 15) étant compris entre 50 et 150 $\mu$m.

Fig.1

Fig.2

Fig.3a

Fig.3b

Fig.3c

Fig.4a

Fig.4b

Fig.5

Fig.6

Fig.7a

Fig.7b

Fig.7c

Fig.8

Fig.9

Fig.10

Fig.11

Fig.12

Fig.13

Fig. 14

EP 3 911 950 B1

34

EP 3 911 950 B1

Fig.15

Fig.16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5985153 A
- WO 2010091874 A
- US 20120003687 A
- US 6204656 A

### Non-patent literature cited in the description

- **S. KARL et al.** *PLOS Neglected Tropical Diseases,* 2013, vol. 7 (5), 2219
- **S. BHAKDI et al.** Optimized high gradient magnetic separation for isolation of Plasmodium-infected red blood cells. *Malaria Journal,* 2010, vol. 9, 38
- **J. NAM et al.** Magnetic Separation of Malaria-Infected Red Blood Cells in Various Developmental Stages. *Anal. Chem.,* 2013, vol. 85, 7316-7323
- **KI-HO HAN ; A. BRUNO FRAZIER.** Paramagnetic capture mode magnetophoretic microseparator for high efficiency blood cell separations. *Lab Chip,* 2006, vol. 6, 265-273
- **E. DU. et al.** Electric Impedance Microflow Cytometry for Characterization of Cell Disease States. *Lab Chip.,* 07 October 2013, vol. 13 (19), 3903-3909
- **M. ; IBRAHIM ; J. CLAUDEL ; D. KOURTICHE ; M. NADI et al.** Geometric parameters optimization of planar interdigitated electrodes for bioimpedance spectroscopy. *J Electr Bioimp,* 2013, vol. 4, 13-22
- **M. GIACOMETTI et al.** *APPLIED PHYSICS LETTERS,* 2018, vol. 113, 203703
- **K. HAN ; A. B. FRAZIER.** *J. Appl. Phys.,* 2004, vol. 96 (10), 5797
- **NAM, JEONGHUN ; HUI HUANG ; HYUNJUNG LIM ; CHAESEUNG LIM ; SEHYUN SHIN.** *Analytical Chemistry,* 2013, vol. 85 (15), 7316-23